# EUROPÄISCHE PATENTSCHRIFT

(11) **EP 1 894 994 B1**
(45) Veröffentlichungstag und Bekanntmachung des Hinweises auf die Patenterteilung: **16.10.2013**
(21) Anmeldenummer: 07016799.4
(22) Anmeldetag: 28.08.2007
(51) Int. Cl.: C11D 17/00, C11D 3/37, A61K 8/03, A61K 8/58, A61Q 19/00

(54) **Zwei- oder mehrphasiges Gesichtsreinigungsmittel mit verbessertem reversiblen Mischungs- und Entmischungsverfahren**
Two or more phase face cleansing agent with improved reversible mixing and demixing solution
Produit de nettoyage du visage à deux phases ou plus doté d'un procédé de mélange et de ségrégation réversible amélioré

(30) Priorität: 31.08.2006 US 824246 P; 01.09.2006 DE 102006041291
(43) Veröffentlichungstag der Anmeldung: 05.03.2008
(73) Patentinhaber: Henkel AG & Co. KGaA, 40589 Düsseldorf (DE)
(72) Erfinder: Hollenbrock, Martina, 40723 Hilden (DE); Kreisig, Annette, 6291 CJ Vaals (NL); Taylor, Tim, Dr. Phoenix, AZ 85013 Arizona (US)

(56) Entgegenhaltungen:
- EP-A- 0 882 442
- WO-A-01/21754
- GB-A- 2 206 048

## Beschreibung

Gegenstand der vorliegenden Erfindung sind unter Normalbedingungen flüssige kosmetische Zusammensetzungen, die im Ruhezustand mindestens zwei voneinander sichtbar getrennte flüssige Phasen enthalten.

Im Ruhestand weisen die erfindungsgemäßen Zusammensetzungen, die Wasser und mindestens eine Ölkomponente enthalten, zwei oder mehr durch eine Phasengrenze getrennte Flüssigkeitsschichten auf, die durch kurze mechanische Agitation, beispielsweise Schütteln oder Rühren des Behälters, zu einer Öl-in-Wasser-Emulsion oder einer Wasser-in-Öl-Emulsion vermischt werden.

Insbesondere betrifft die vorliegende Erfindung wässrige, mindestens ein Öl und mindestens einen Emulgator enthaltende, flüssige, visuell mindestens zweischichtige Zusammensetzungen, die mindestens eine kontinuierliche wässrige Phase I und mindestens eine diskontinuierliche ölige Phase II aufweisen, wobei die ölige Phase II zusammen mit einem Teil einer wässrigen Phase I eine Emulsionsschicht, und ein weiterer Teil der wässrigen Phase I eine wässrige Schicht der Zusammensetzungen bildet, die Zusammensetzungen sich durch Schütteln temporär in visuell homogene Emulsionen überführen lassen und weiterhin mindestens ein wasserlösliches Polymer enthalten.

Der Verbraucher erwartet bei einem Reinigungs- oder Pflegemittel, insbesondere wenn es sich um ein kosmetisches Mittel handelt, in der Regel nicht nur eine funktionale Wirkung, sondern in gleicher Weise auch ein attraktives Aussehen und eine die Wirkung unterstreichende galenische Zubereitung des Mittels.

In den letzten Jahren fanden mehrphasige Zubereitungen, die im Ruhezustand in Form mehrerer getrennter Phasen bzw. Schichten vorliegen, in Verbraucherkreisen ein zunehmendes Interesse. Diese Phasentrennung bietet einerseits den technischen Vorteil einer größeren Formulierungsfreiheit hinsichtlich ansonsten miteinander unzureichend kompatibler Rezepturbestandteile, und andererseits die Möglichkeit, der Formulierung bestimmte optische bzw. ästhetische Effekte zu verleihen. So können die unterschiedlichen Phasen beispielsweise unterschiedlich gefärbt werden, und insbesondere können eine klare und eine milchige Phase, nämlich die Emulsionsphase, miteinander kombiniert werden. Ein derartiges Erscheinungsbild ist beispielsweise besonders gut geeignet, dem Verbraucher unterschiedliche Eigenschaften visuell zu vermitteln, was vor allem bei den sogenannten "two-in-one"-Produkten eine Rolle spielt.

Andererseits muss es dem Verbraucher möglich sein, eine solche Zubereitung ohne besonderen Aufwand aus der Verpackung, insbesondere einer Flasche, so entnehmen zu können, dass die Zusammensetzung der entnommenen Probe identisch ist mit der Zusammensetzung der gesamten Zubereitung. Dies kann am einfachsten gewährleistet werden, indem die mehrphasige Zubereitung durch kurzes Schütteln in eine makroskopisch einheitliche Form, z. B. eine einheitlich aussehende Emulsion, überführt wird. Diese soll sich, vor allem aus optischen bzw. ästhetischen Gründen, im Ruhezustand so rasch wieder in die ursprüngliche mehrphasige beziehungsweise mehrschichtige Form zurückwandeln, dass der Verbraucher auch bei kurz aufeinander folgenden Anwendungen eines solchen Produktes dieses jeweils wieder im entmischten Zustand vorfindet.

### Stand der Technik

Das Dokument JP-A 60078907 beschreibt zweischichtige kosmetische Zubereitungen mit guter Lagerstabilität, die als wesentliche Komponenten ein bei gewöhnlicher Temperatur flüssiges Öl und ein nichtionisches Tensid enthalten, und die aus einer oberen emulgierten Schicht und einer unteren wässrigen Schicht bestehen.

In dem Dokument JP-A 62263297 ist eine Waschmittelzubereitung beschrieben, die ein Tensid und ein wasserlösliches Polymer enthält. Beim Schütteln der Zusammensetzung bildet sich eine einheitlich aussehende Flüssigkeit, die sich beim Stehen in zwei flüssige Phasen auftrennt.

Die deutsche Patentanmeldung DE 19915837 beschreibt wässrige Zubereitungen, die im Ruhezustand in Form von zwei oder mehr entmischten, wässrigen, unter Bewegung zeitweise ineinander dispergierbaren Phasen vorliegen. Die Zubereitungen enthalten wenigstens eine gelöste, oberflächenaktive Verbindung und wenigstens eine gelöste Polymerverbindung, sowie zur Beschleunigung der Phasentrennung im Ruhezustand wenigstens einen wasserlöslichen ein- oder mehrwertigen Alkohol.

GB 2206048 A1 offenbart zweischichtige Zusammensetzungen mit einer Siliconöl-haltigen Ölphasenschicht und einer Wasserphasenschicht ohne einen Gehalt an verdickendem Polymer, die im Ruhezustand vollständig getrennt voneinander vorliegen.

WO 01/21754 A1 offenbart zweischichtige Reinigungsmittel mit 0,01 - 10 Gew.-% Aniontensiden und/oder 0,002 - 8 Gew.-% nichtionischen Tensiden und, optional, 0 - 0,5 Gew.-% verdickendem Polymer, bei denen im Ruhezustand eine Schicht als Emulsion vorliegt.

EP 882442 A1 offenbart kosmetische Zusammensetzungen, die im Ruhezustand jeweils eine wässrige Schicht, eine Polyolschicht und eine Ölschicht mit stabilen Grenzflächen ausbilden. Keine der Schichten liegt im Ruhezustand als Emulsion vor.

Die aus dem Stand der Technik bekannten Zusammensetzungen ermöglichen zwar die Formulierung von zweischichtigen Systemen aus einer wässrigen Schicht und einer Emulsionsschicht, die beim Schütteln eine einheitlich aussehende Flüssigkeit bilden. Lässt man diese jedoch nach dem Schütteln ruhen, so erfolgt eine sehr langsame Phasentrennung, die typischerweise einen Zeitbedarf in der Größenordnung eines Tages benötigt. Selbst dann ist jedoch in vielen Fällen die wässrige Schicht, die eigentlich emulsionsfrei sein soll, immer noch trüb, und zwischen den beiden Schichten bildet sich keine scharfe Grenze aus, was ein optisch unbefriedigendes Bild bietet. Zudem kommt es in vielen Fällen beim Stehen dieser Zusammensetzungen zu einer teilweisen Koaleszenz der emulgierten Tröpfchen, was eine unerwünschte Bildung von "Fettaugen" oder sogar die Abscheidung einer (reinen) Ölschicht auf der Oberfläche der Zusammensetzungen zur Folge hat.

Untersuchungen der Erfinder der vorliegenden Anmeldung zeigten, dass es bei der Formulierung derartiger Zusammensetzungen einen Zielkonflikt gibt zwischen der Stabilität der Emulsionsphase gegenüber einer Koagulation und der Geschwindigkeit der Phasentrennung nach dem Schütteln. Dies bedeutet, dass Zusammensetzungen mit einer hohen Stabilität der Emulsionsphase den Nachteil einer sehr langsamen Phasentrennung nach dem Schütteln aufweisen. Bei einer zu geringen Stabilität der Emulsionsphase trennt sich diese nach einer gewissen Ruhezeit in reine Öl- und Wasserphasen auf.

Zur Verwirklichung einer gleichbleibend effektiven Wirkung eines Zwei- oder Mehrphasenmittels ist einerseits die homogene Mischung der Phasen vor der Anwendung, sowie andererseits die Reversibilität dieser Mischung entscheidend. Allerdings ist in den mehrphasigen Mitteln des Standes der Technik das Misch- und das Entmischungsverhalten der Phasen nach der Anwendung nicht befriedigend. Insbesondere bei mehrmalig ausgeführtem Misch- und Entmischvorgang ist die vollständige Entmischung der Phasen nicht zufriedenstellend gewährleistet.
Es hat daher in der Vergangenheit nicht an Versuchen gefehlt, hier für Abhilfe zu sorgen.

### Aufgabenstellung

Es bestand daher weiterhin die Aufgabe, ein Mittel zu finden, bei dem die genannten unerwünschten Nachteile weiter reduziert oder ganz ausgeschlossen werden.
Der vorliegenden Erfindung lag somit das Problem zugrunde, eine lagerstabile, im Ruhezustand visuell mindestens zweischichtig erscheinende Zusammensetzung zur Verfügung zu stellen, wobei mindestens eine Schicht klar sein sollte, mindestens eine weitere Schicht jedoch eine milchig erscheinende Emulsion darstellen sollte. Diese Zusammensetzung sollte sich durch Schütteln in eine einheitlich aussehende Dispersion überführen lassen, welche sich beim Stehen relativ rasch, insbesondere innerhalb eines Zeitraums von der Größenordnung einer Stunde, wieder in die ursprünglich vorhandenen Schichten auftrennt. Dabei war es zusätzlich wünschenswert, dass sich die ursprüngliche Klarheit der wässrigen Schicht nach einer Ruhezeit wieder einstellt, und dass die klare und die Emulsionsschicht wieder optisch deutlich voneinander abgegrenzt waren. Die Zusammensetzung sollte weiterhin eine gute Lagerstabilität aufweisen, worunter insbesondere eine hohe Stabilität der Emulsionsschicht bzw. der Emulsionsphase gegenüber einer Koaleszenz der emulgierten Tröpfchen zu verstehen ist. Die gute Lagerstabilität sollte insbesondere auch für eine Lagerung bei höheren Temperaturen, insbesondere bei Temperaturen von 40 °C und mehr, bestehen.
Eine weitere Aufgabe war es, besonders augen- und schleimhautverträgliche kosmetische Zusammensetzungen bereitzustellen, die zur Reinigung der Gesichtshaut, insbesondere im besonders empfindlichen Augenbereich, geeignet sind.

Um ein ansprechendes optisches Erscheinungsbild der Formulierung zu erzielen, ist es wünschenswert, dass sich die Schichten schnell und möglichst vollständig wieder zu mindestens einer wässrigen Schicht und mindestens einer Emulsionsschicht entmischen, sobald sie im Ruhezustand sind. Die Zeit bis zur vollständigen Entmischung reicht dabei von wenigen Sekunden bis zu 24 Stunden, bevorzugt von 30 Sekunden bis 2 Stunden, besonders bevorzugt von 60 Sekunden bis 60 Minuten.

Zweiphasige kosmetische Zusammensetzungen mit nicht dauerhaft emulgierter Öl- und Wasserphase sind im Stand der Technik bekannt. Es zeigt sich, dass insbesondere die Faktoren Emulgatormenge und -typ, Gewichtsverhältnis zwischen Öl- und Wasserphase, gegebenenfalls ein Elektrolytgehalt. Verdickergehalt und gegebenenfalls die Anwesenheit von Alkoholen die Eigenschaften derartiger Zusammensetzungen, vor allem die Geschwindigkeit der Phasentrennung, beeinflussen. Bevorzugt enthalten die erfindungsgemäßen Zusammensetzungen möglichst wenig Elektrolyten und möglichst wenig Alkohole, insbesondere möglichst wenig einwertige Alkohole wie Ethanol oder Isopropanol, da diese die Augen und Schleimhäute reizen können.
Eine weitere Aufgabe war die Verbesserung der Reinigungsleistung mehrphasiger bzw. mehrschichtiger Zusammensetzungen, die eine wässrige Schicht und eine Emulsionsschicht enthalten.
Über die Zweiphasigkeit lassen sich verschiedene optische Effekte einstellen. Das Sichtbarmachen der Ölphase gegenüber einer Wasserphase unterstreicht optisch für den Verbraucher den pflegenden Effekt derartiger Zusammensetzungen. Durch die Wahl öllöslicher bzw. wasserlöslicher Farbstoffe kann die optische Unterscheidung der beiden Phasen betont werden. Bei der kurzzeitigen Emulgierung bilden sich Mischfarben, die sich bei der anschließenden Phasentrennung durch das Entmischen effektvoll verändern. Die Auswahl der einzelnen Bestandteile der Zusammensetzung kann auch so erfolgen, dass während des Entmischungsvorgangs für eine gewisse Zeit eine intermediäre dritte Emulsionsschicht als Phasengrenze zwischen Öl- und Wasserphase bestehen bleibt. Ästhetisch weniger ansprechende Phänomene, z.B. Tropfen- oder Schlierenbildung an der Innenwand des Aufbewahrungsbehälters, sollten durch geeignete Abstimmung der einzelnen Bestandteile aufeinander vermieden werden.

Ein erster Gegenstand der vorliegenden Erfindung ist eine wässrige, mindestens ein Öl und mindestens einen Emulgator oder mindestens ein Tensid enthaltende, flüssige, mindestens zweischichtige Zusammensetzung, die mindestens eine kontinuierliche wässrige Phase I und mindestens eine diskontinuierliche ölige Phase II aufweist, wobei im Ruhezustand die ölige Phase II zusammen mit einem Teil einer wässrigen Phase I eine Emulsionsschicht, und ein weiterer Teil der wässrigen phase I eine wässrige Schicht der Zusammensetzung bildet, und die Zusammensetzung sich durch mechanische Bewegung reversibel temporär in eine Emulsion überführen lässt, dadurch gekennzeichnet, dass die ölige Phase II mindestens ein bei Normalbedingungen flüssiges Siliconöl enthält, der Wassergehalt 50 - 85 Gew.-%, bevorzugt 60 - 75 Gew.-% und besonders bevorzugt 65 - 70 Gew.-%, jeweils bezogen auf die gesamte Zusammensetzung, beträgt, der gesamte Emulgator- und/oder Tensidgehalt 0,01 bis weniger als 0,1 Gew.-% beträgt, jeweils bezogen auf die gesamte Zusammensetzung, und mindestens ein verdickendes Polymer in einer Gesamtmenge von 0,1 - 2 Gew.-%, bevorzugt 0,3 - 1 Gew.-% und besonders bevorzugt 0,3 - 0,5 Gew.-%, jeweils bezogen auf die gesamte Zusammensetzung, enthalten ist.
Die erfindungsgemäßen Zusammensetzungen entfalten ihre volle Wirkung am besten, wenn sie in homogener = vollständig durchmischter Form aufgebracht werden. Dazu werden die Zusammensetzungen durch mechanische Einwirkung, z. B. einfaches Schütteln des sie enthaltenden Behälters mit der Hand, reversibel in ein homogen durchmischtes System überführt. Um ein homogenes Auftragen sicherzustellen, muss dieser homogen durchmischte Zustand für eine ausreichende Zeit erhalten bleiben, bevor sich die einzelnen Phasen wieder ausbilden. Für die erfindungsgemäße Lehre hat es sich als ausreichend erwiesen, wenn dieser homogene Zustand mindestens 20 Sekunden, insbesondere mindestens 30 Sekunden, stabil ist, bevor für den Betrachter wieder eine Grenzschicht und somit die Ausbildung der einzelnen Phasen erkennbar wird. Nach einem gewissen Zeitraum, bevorzugt von höchstens 60 Minuten, besonders bevorzugt von höchstens 30 Minuten, hat sich die reversible Mischung der Phasen durch Entmischung vollzogen, so dass der Ausgangszustand unter Erhalt des Zwei- oder Mehrphasensystems wieder erreicht wird.
Erfindungsgemäß bedeutet der Begriff "flüssig", dass die entsprechende Substanz unter Normalbedingungen, das heißt, bei 20 °C und 1 Atmosphäre Luftdruck, flüssig vorliegt.
Die erfindungsgemäßen Zusammensetzungen enthalten neben Wasser zwingend mindestens ein flüssiges Siliconöl als Ölkomponente, wodurch sie sich von Zusammensetzungen des Standes der Technik unterscheiden. Überraschend wurde festgestellt, dass die Auswahl eines Siliconöls positive Auswirkungen auf die Emulgierung und anschließende Rückbildung der Emulsionsschicht und der Wasserschicht, die durch eine mit bloßem Auge makroskopisch sichtbare Phasengrenze voneinander getrennt sind, hat. Die Emulgierung nach der kurzzeitigen mechanischen Agitation verläuft schnell und ergibt eine gleichmäßige Emulsion. Auch die anschließende Wiederherstellung der makroskopischen Phasengrenze erfolgt in einem überschaubaren Zeitraum, das heißt, nach ca. 30 bis 60 Minuten im Ruhezustand hat sich die Emulsionsschicht von der Wasserschicht getrennt.

In einer bevorzugten Ausführungsform der Erfindung bildet im Ruhezustand die Emulsionsschicht die obere Schicht, während die Wasserschicht die untere Schicht bildet.

Eine bevorzugte Ausführungsform der Erfindung ist dadurch gekennzeichnet, dass das mindestens eine flüssige Siliconöl ausgewählt ist unter:
(i) Polyalkylsiloxanen, Polyarylsiloxanen, Polyalkylarylsiloxanen, die flüchtig oder nicht flüchtig, geradkettig, verzweigt oder cyclisch, vernetzt oder nicht vernetzt sind;
(ii) Polysiloxanen, die in ihrer allgemeinen Struktur eine oder mehrere organofunktionelle Gruppen enthalten, die ausgewählt sind unter:
   a) substituierten oder unsubstituierten aminierten Gruppen;
   b) (per)fluorierten Gruppen;
   c) Thiolgruppen;
   d) Carboxylatgruppen;
   e) hydroxylierten Gruppen;
   f) Acyloxyalkylgruppen;
   g) amphoteren Gruppen;
   h) Hydroxyacylaminogruppen;
(iii) gepfropften Siliconpolymeren mit nicht siliconhaltigem, organischen Grundgerüst, die aus einer organischen Hauptkette bestehen, welche aus organischen Monomeren gebildet wird, die kein Silicon enthalten, auf die in der Kette sowie gegebenenfalls an mindestens einem Kettenende mindestens ein Polysiloxanmakromer gepfropft wurde;
(iv) gepfropften Siliconpolymeren mit Polysiloxan- Grundgerüst, auf das nicht siliconhaltige, organische Monomere gepfropft wurden, die eine Polysiloxan-Hauptkette aufweisen, auf die in der Kette sowie gegebenenfalls an mindestens einem ihrer Enden mindestens ein organisches Makromer gepfropft wurde, das kein Silicon enthält;
(v) oder deren Gemischen.

### Lineare Polydimethylsiloxane

Besonders bevorzugte erfindungsgemäße kosmetische oder dermatologische Zubereitungen sind dadurch gekennzeichnet, dass sie mindestens ein Silicon der Formel (I)

(CH₃)₃Si-[O-Si(CH₃)₂]ₓ-O-Si(CH₃)₃ (I),

enthalten, in der x für eine Zahl von 0 bis 100, vorzugsweise von 1 bis 50, weiter bevorzugt von 2 bis 20 und insbesondere 3 bis 10, steht.

Die erfindungsgemäß bevorzugten kosmetischen oder dermatologischen Zubereitungen enthalten ein Silicon der vorstehenden Formel I. Diese Silicone werden nach der INCI-Nomenklatur als DIMETHICONE bezeichnet. Es werden im Rahmen der vorliegenden Erfindung als Silicon der Formel I vorzugsweise die Verbindungen:

(CH₃)₃Si-O-Si(CH₃)₃

(CH₃)₃Si-O-(CH₃)₂Si-O-Si(CH₃)₃

(CH₃)₃Si-[O-(CH₃)₂Si]₂-O-Si(CH₃)₃

(CH₃)₃Si-[O-(CH₃)₂Si]₃-O-Si(CH₃)₃

(CH₃)₃Si-[O-(CH₃)₂Si]₄-O-Si(CH₃)₃

(CH₃)₃Si-[O-(CH₃)₂Si]₅-O-Si(CH₃)₃

(CH₃)₃Si-[O-(CH₃)₂Si]₆-O-Si(CH₃)₃

(CH₃)Si-[O-(CH₃)₂Si]₇-O-Si(CH₃)₃

(CH₃)₃Si-[O-(CH₃)₂Si]₈-O-Si(CH₃)₃

(CH₃)₃Si-[O-(CH₃)₂Si]a-O-Si(CH₃)₃

(CH₃)₃Si-[O-(CH₃)₂Si]₁₀-O-Si(CH₃)₃

(CH₃)₃Si-[O-(CH₃)₂Si]₁₁-O-Si(CH₃)₃

(CH₃)₃Si-[O-(CH₃)₂Si]₁₂-O-Si(CH₃)₃

(CH₃)₃Si-[O-(CH₃)₂Si]₁₃-O-Si(CH₃)₃

(CH₃)₃Si-[O-(CH₃)₂Si]₁₄-O-Si(CH₃)₃

(CH₃)₃Si-[O-(CH₃)₂Si]₁₅-O-Si(CH₃)₃

(CH₃)₃Si-[O-(CH₃)₂Si]₁₆-O-Si(CH₃)₃

(CH₃)₃Si-[O-(CH₃)₂Si]₁₇-O-Si(CH₃)₃

(CH₃)₃Si-[O-(CH₃)₂Si]₁₈-O-Si(CH₃)₃

(CH₃)₃Si-[O-(CH₃)₂Si]₁₉-O-Si(CH₃)₃

(CH₃)₃Si-[O-(CH₃)₂Si]₂₀-O-Si(CH₃)₃

eingesetzt, wobei (CH₃)₃Si--O-Si(CH₃)₃, (CH₃)₃Si-O-(CH₃)₂Si-O-Si(CH₃)₃ und/oder (CH₃)₃Si-[O-(CH₃)₂Si]₂-O-Si(CH₃)₃ besonders bevorzugt sind.

Selbstverständlich können auch Mischungen der oben genannten Silicone in den erfindungsgemäßen Zusammensetzungen enthalten sein.
Bevorzugte erfindungsgemäß einsetzbare Dimethicone weisen bei 25°C Viskositäten von 0,2 - 50000 mm²s⁻¹, bevorzugt von 0,5 bis 10000 mm²s⁻¹, besonders bevorzugt von 1 - 350 und außerordentlich bevorzugt von 1,5 - 10 - 20 - 50 mm²s⁻¹ auf.
Beispiele für erfindungsgemäß bevorzugte Dimethicone sind Hexamethyldisiloxan (L₂), Octamethyltrisiloxan (L₃), Decamethyltetrasiloxan (L₄), beliebige Zweier- und Dreiermischungen aus L₂, L₃ und/oder L₄, wie sie z. B. in den Handelsprodukten DC 2-1184, Dow Corning^{®} 200 (0, 65 cSt) und Dow Corning^{®} 200 (1,5 cSt) von Dow Coming enthalten sind, weiterhin aus nichtflüchtigen höhermolekularen linearen Dimethylpolysiloxanen, im Handel erhältlich z. B. unter der Bezeichnung Dow Corning^{®} 190, Baysilon^{®} 350 M und diverser Produkte aus der Serie Dow Corning^{®} 200 Fluid mit kinematischen Viskositäten im Bereich von 5 - 50000 cSt, bevorzugt 5 - 10000 cSt, besonders bevorzugt 10 - 350 cSt und außerordentlich bevorzugt 50 - 100 cSt, gemessen bei 25 °C. Ganz besonders bevorzugt sind lineare Dimethylpolysiloxane mit kinematischen Viskositäten bis zu 10.000 cSt, gemessen bei 25 °C. Die Bestimmung der Viskositäten erfolgt dabei nach der Kugelfallmethode entsprechend der Methode "British Standard 188". Vergleichbare Werte werden mit zum "British Standard 188" analogen Prüfvorschriften der Hersteller erhalten, beispielsweise der "CTM 0577" der Dow Corning Corporation.

Weitere bevorzugte Silicone sind ausgewählt aus den Alkylmethylpolysiloxanen, insbesondere solchen mit der allgemeinen Formel (I-Alkyl),

(CH₃)₃Si-[O-SiR(CH₃)]ₓ-O-Si(CH₃)₃ (I-Alkyl),

wobei R für einen C₂₋₂₄ Alkyl-Rest steht, insbesondere für Ethyl, Propyl, Butyl, Pentyl, Hexyl, Heptyl, Octyl, 2-Ethylhexyl, Nonyl, Decyl, Undecyl, Dodecyl, Lauryl, Tridecyl, Tetradecyl, Myristyl, Pentadecyl, Hexadecyl, Cetyl, Isocetyl, Heptadecyl, Octadecyl, Stearyl, Isostearyl, Eicosanyl, Arachidyl, Behenyl, Erucyl, Brassidyl,
und x für eine Zahl von 1 bis 100, vorzugsweise von 2 bis 50, weiter bevorzugt von 3 bis 20 und insbesondere 4 bis 10, steht.
Erfindungsgemäß besonders bevorzugte Silicone der Formel (I-Alkyl) sind C₂₋₂₄-Alkyl-Methicone, wie beispielsweise die 3-C₂₀₋₂₄-Alkylderivate des 1,1,1,3,5,5,5-Heptamethyl-Trisiloxans, besonders bevorzugt jeweils mit Viskositäten im Bereich von 5 - 50000 cSt (bei 25 °C).
Erfindungsgemäß besonders bevorzugte Silicone der Formel (I-Alkyl) sind Ethyl Trisiloxane, (3-Ethylheptamethyltrisiloxan), Hexyl Trisiloxane, (3-Hexylheptamethyltrisiloxan), Lauryl Trisiloxane, (3-Laurylheptamethyltrisiloxan), Myristyl Trisiloxane, (3-Myristylheptamethyltrisiloxan) und C₂₀₋₂₄Alkyl-Methicone.

Weitere bevorzugte Silicone sind ausgewählt aus den Arylmethylpolysiloxanen, insbesondere solchen mit der allgemeinen Formel (I-Aryl), 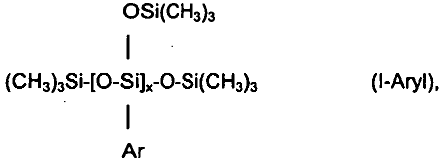 wobei Ar für einen aromatischen Rest steht, insbesondere für Phenyl, Benzyl, Phenylmethyl, Phenylethyl, Phenylpropyl, Phenylbutyl, besonders bevorzugt für Phenyl,
und x für eine Zahl von 1 bis 50, vorzugsweise von 2 bis 30, weiter bevorzugt von 3 bis 10 und insbesondere 4 bis 5, steht.
Erfindungsgemäß besonders bevorzugte Silicone der Formel (I-Aryl) sind die Phenyl Trimethicone mit Ar = Ph und x = 1, Ar = Ph und x = 2 und Ar = Ph und x = 3.

### Aminofunktionelle Silicone

Besonders bevorzugte erfindungsgemäße Mittel, insbesondere solche, die zur Haarbehandlung angewendet werden, enthalten ein oder mehrere aminofunktionelle Silicone. Solche Silicone können z.B. durch die Formel

M(RₐQ_{b}SiO_{(4-a-b)/2)x}(R_{c}SiO_{(4-c)/2})_{y}M

beschrieben werden, wobei in der obigen Formel R ein Kohlenwasserstoff oder ein Kohlenwasserstoffrest mit 1 bis etwa 6 Kohlenstoffatomen ist, Q ein polarer Rest der allgemeinen Formel -R¹HZ ist, worin R¹ eine zweiwertige, verbindende Gruppe ist, die an Wasserstoff und den Rest Z gebunden ist, zusammengesetzt aus Kohlenstoff- und Wasserstoffatomen, Kohlenstoff-, Wasserstoff- und Sauerstoffatomen oder Kohlenstoff-, Wasserstoff- und Stickstoffatomen, und Z ein organischer, aminofunktioneller Rest ist, der mindestens eine aminofunktionelle Gruppe enthält; "a" Werte im Bereich von etwa 0 bis etwa 2 annimmt, "b" Werte im Bereich von etwa 1 bis etwa 3 annimmt, "a" + "b" kleiner als oder gleich 3 ist, und "c" eine Zahl im Bereich von etwa 1 bis etwa 3 ist, und x eine Zahl im Bereich von 1 bis etwa 2.000, vorzugsweise von etwa 3 bis etwa 50 und besonders bevorzugt von etwa 3 bis etwa 25 ist, und y eine Zahl im Bereich von etwa 20 bis etwa 10.000, vorzugsweise von etwa 125 bis etwa 10.000 und besonders bevorzugt von etwa 150 bis etwa 1.000 ist, und M eine geeignete Silicon-Endgruppe ist, wie sie im Stand der Technik bekannt ist, vorzugsweise Trimethylsiloxy. Nicht einschränkende Beispiele der durch R repräsentierten Reste schließen Alkylreste, wie Methyl, Ethyl, Propyl, Isopropyl, Isopropyl, Butyl, Isobutyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3-Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R ein Alkylrest, der 1 bis etwa 6 Kohlenstoffatomen enthält, und besonders bevorzugt ist R Methyl. Beispiele von R¹ schließen Methylen, Ethylen, Propylen, Hexamethylen, Decamethylen, -CH₂CH(CH₃)CH₂-, Phenylen, Naphthylen, -CH₂CH₂SCH₂CH₂-, -CH₂CH₂OCH₂-, -OCH₂CH₂-, -OCH₂CH₂CH₂-, -CH₂CH(CH₃)C(O)OCH₂-, -(CH₂)₃CC(O)OCH₂CH₂-, -C₆H₄C₆H₄-, -C₆H₄CH₂C₆H₄-; und -(CH₂)₃C(O)SCH₂CH₂- ein.

Z ist ein organischer, aminofunktioneller Rest, enthaltend mindestens eine funktionelle Aminogruppe. Eine mögliche Formel für Z ist NH(CH₂)_{z}NH₂, worin z 1 oder mehr ist. Eine andere mögliche Formel für Z ist -NH(CH₂)_{z}(CH₂)_{zz}NH, worin sowohl z als auch zz unabhängig 1 oder mehr sind, wobei diese Struktur Diamino-Ringstrukturen umfasst, wie Piperazinyl. Z ist besonders bevorzugt ein -NHCH₂CH₂NH₂-Rest. Eine andere mögliche Formel für Z ist -N(CH₂)_{z}(CH₂)_{zz}NX₂ oder -NX₂, worin jedes X von X₂ unabhängig ausgewählt ist aus der Gruppe, bestehend aus Wasserstoff und Alkylgruppen mit 1 bis 12 Kohlenstoffatomen, und zz 0 ist.

Q ist besonders bevorzugt ein polarer, aminfunktioneller Rest der Formel -CH₂CH₂CH₂NHCH₂CH₂NH₂. In den Formeln nimmt "a" Werte im Bereich von etwa 0 bis etwa 2 an, "b" nimmt Werte im Bereich von etwa 2 bis etwa 3 an, "a" + "b" ist kleiner als oder gleich 3, und "c" ist eine Zahl im Bereich von etwa 1 bis etwa 3. Das molare Verhältnis der RₐQ_{b} SiO_{(4-a-b)/2}-Einheiten zu den R_{c}SiO_{(4-c)/2}-Einheiten liegt im Bereich von etwa 1 : 2 bis 1 : 65, vorzugsweise von etwa 1 : 5 bis etwa 1 : 65 und besonders bevorzugt von etwa 1 : 15 bis etwa 1 : 20. Werden ein oder mehrere Silicone der obigen Formel eingesetzt, dann können die verschiedenen variablen Substituenten in der obigen Formel bei den verschiedenen Siliconkomponenten, die in der Siliconmischung vorhanden sind, verschieden sein.

Bevorzugte erfindungsgemäße kosmetische oder dermatologische Zubereitungen enthalten ein aminofunktionelles Silicon der Formel (II)

R'ₐG₃₋ₐ-Si(OSiG₂)ₙ-(OSiG_{b}R'_{2-b)m}-O-SiG₃₋ₐ-R'ₐ (II)

worin bedeutet:
- G kann sein: -H, eine Phenylgruppe, -OH, -O-CH₃, -CH₃, -O-CH₂CH₃, -CH₂CH₃, -O-CH₂CH₂CH₃,-CH₂CH₂CH₃, -O-CH(CH₃)₂, -CH(CH₃)₂, -O-CH₂CH₂CH₂CH₃, -CH₂CH₂CH₂CH₃, -O-CH₂CH(CH₃)₂, -CH₂CH(CH₃)₂, -O-CH(CH₃)CH₂CH₃, -CH(CH₃)CH₂CH₃, -O-C(CH₃)₃, -C(CH₃)₃;
- a steht für eine Zahl zwischen 0 und 3, insbesondere 0;
- b steht für eine Zahl zwischen 0 und 1, insbesondere 1,
- m und n sind Zahlen, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt,
- R' ist ein monovalenter Rest, ausgewählt aus
   o -Q-N(R")-CH₂-CH₂-N(R")₂
   o -Q-N(R")₂
   o -Q-N⁺(R")₃A⁻
   o -Q-N⁺H(R")₂ A⁻
   o -Q-N⁺H₂(R")A⁻
   o -Q-N(R")-CH₂-CH₂-N⁺R"H₂A⁻,
wobei jedes Q für eine chemische Bindung, -CH₂-, -CH₂-CH₂-, -CH₂CH₂CH₂-, -(CH₃)₂-, -CH₂CH₂CH₂CH₂-, -CH₂C(CH₃)₂-, -CH(CH₃)CH₂CH₂- steht,
R" für gleiche oder verschiedene Reste aus der Gruppe -H, -Phenyl, -Benzyl, -CH₂-CH(CH₃)Ph, der C₁₋₂₀-Alkylreste, vorzugsweise -CH₃, -CH₂CH₃, -CH₂CH₂CH₃, -CH(CH₃)₂, -CH₂CH₂CH₂H₃, -CH₂CH(CH₃)₂, -CH(CH₃)CH₂CH₃, -C(CH₃)₃, steht und A ein Anion repräsentiert, as vorzugsweise ausgewählt ist aus Chlorid, Bromid, lodid oder Methosulfat.

Besonders bevorzugte erfindungsgemäße kosmetische oder dermatologische Zubereitungen sind dadurch gekennzeichnet, dass sie mindestens es ein aminofunktionelles Silicon der Formel (IIa) 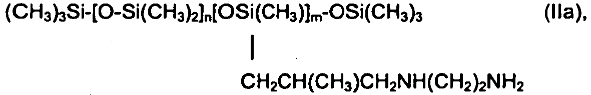 enthalten, worin m und n Zahlen sind, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt.

Diese Silicone werden nach der INCI-Deklaration als Trimethylsilylamodimethicone bezeichnet.

Besonders bevorzugt sind auch erfindungsgemäße kosmetische oder dermatologische Zubereitungen, die mindestens ein aminofunktionelles Silicon der Formel (IIb) 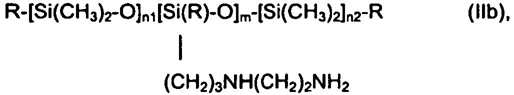 enthalten, worin R für -OH, -O-CH₃ oder eine -CH₃-Gruppe steht und m, n1 und n2 Zahlen sind, deren Summe (m + n1 + n2) zwischen 1 und 2000, vorzugsweise zwischen 50 und 150 liegt, wobei die Summe (n1 + n2) vorzugsweise Werte von 0 bis 1999 und insbesondere von 49 bis 149 und m vorzugsweise Werte von 1 bis 2000, insbesondere von 1 bis 10 annimmt.

Diese Silicone werden nach der INCI-Deklaration als Amodimethicone bezeichnet.

Unabhängig davon, welche aminofunktionellen Silicone eingesetzt werden, sind erfindungsgemäße kosmetische oder dermatologische Zubereitungen bevorzugt, die ein aminofunktionelles Silicon enthalten, dessen Aminzahl oberhalb von 0,25 meq/g, vorzugsweise oberhalb von 0,3 meq/g und insbesondere oberhalb von 0,4 meq/g liegt. Die Aminzahl steht dabei für die Milli-Äquivalente Amin pro Gramm des aminofunktionellen Silicons. Sie kann durch Titration ermittelt und auch in der Einheit mg KOH/g angegeben werden.

Erfindungsgemäß bevorzugte kosmetische oder dermatologische Zubereitungen sind dadurch gekennzeichnet, dass sie, bezogen auf ihr Gewicht, 0,01 bis 10 Gew.-%, vorzugsweise 0,1 bis 8 Gew.-%, besonders bevorzugt 0,25 bis 7,5 Gew.-% und insbesondere 0,5 bis 5 Gew.-% aminofunktionelle(s) Silicon(e) enthalten.

### Cyclomethicone

Auch die nach INCI als CYCLOMETHICONE bezeichneten cyclischen Dimethicone sind erfindungsgemäß bevorzugt einsetzbar. Hier sind erfindungsgemäße kosmetische oder dermatologische Zubereitungen bevorzugt, die mindestens ein Silicon der Formel. III 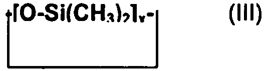 enthalten, in der x für eine Zahl von 3 bis 200, vorzugsweise von 3 bis 10, weiter bevorzugt von 3 bis 7 und insbesondere 3, 4, 5 oder 6, steht.
Beispiele für bevorzugte cyclische Siliconöle sind Octamethylcyclotetrasiloxan, Decamethylcyclopentasiloxan und Dodecamethylcyclohexasiloxan sowie Mischungen hiervon, wie sie z. B. in den Handelsprodukten DC 244, 245, 344 und 345 von Dow Coming enthalten sind.

Die vorstehend beschriebenen nicht-cyclischen Silicone weisen ein Rückgrat auf, das aus -Si-O-Si-Einheiten aufgebaut ist. Selbstverständlich können diese Si-O-Si-Einheiten auch durch Kohlenstoffketten unterbrochen sein. Entsprechende Moleküle sind durch Kettenverlängerungsreaktionen zugänglich.
Kettenverlängerungsreaktionen mit Polysiloxanen sind bekannt und können beispielsweise die Hydrosilylierungsreaktion umfassen, in der eine Si-H Gruppe mit einer aliphatisch ungesättigten Gruppe in Gegenwart eines Platin/Rhodium-Katalysators unter Bildung von Polysiloxanen mit einigen Si-(C)ₚ-Si Bindungen (p = 1-6) reagiert, wobei die Polysiloxane auch als Polysiloxane-Polysilalkylene-Copolymere bezeichnet werden.

Die Kettenverlängerungsreaktion kann auch die Reaktion einer Si-OH Gruppe (beispielsweise eines Hydroxy-terminierten Polysiloxans) mit einer Alkoxygruppe (beispielsweise Alkoxysilanen, Silikaten oder Alkoxysiloxanen) in Gegenwart eines metallhaltigen Katalysators unter Bildung von Polysiloxanen umfassen.
Die Polysiloxane, die in der Kettenverlängerungsreaktion eingesetzt werden, umfassen ein substantiell lineares Polymer der folgenden Struktur:

R-Si(R₂)-[-O-Si(R₂)-]ₙ-O-SiR₃

In dieser Struktur steht jedes R unabhängig voneinander für einen Kohlenwasserstoffrest mit bis zu 20 Kohlenstoffatomen, vorzugsweise mit 1 bis 6 C-Atomen, wie beispielsweise einer Alkylgruppe (beispielsweise Methyl, Ethyl, Propyl oder Butyl), eine Arylgruppe (beispielsweise Phenyl), oder die für die Kettenverlängerungsreaktion benötigte Gruppe ("reaktive Gruppe", beispielsweise Si-gebundene H-Atome, aliphatisch ungesättigte Gruppen wie Vinyl, Allyl oder Hexenyl, Hydroxy, Alkoxy wie Methoxy, Ethoxy oder Propoxy, Alkoxy-Alkoxy, Acetoxy, Amino usw.), mit der Maßgabe, dass durchschnittlich ein bis zwei reaktive Gruppen pro Polymer vorliegen, n ist eine positive Zahl > 1. Vorzugsweise ist eine Mehrzahl der reaktiven Gruppen, besonders bevorzugt > 90%, und insbesondere > 98% der reaktiven Gruppen, an den endständigen Si-Atomen im Siloxan gebunden. Vorzugsweise steht n für Zahlen, die Polysiloxane beschreiben, welche Viskositäten von 1 - 1.000.000 mm²/s besitzen, bevorzugt Viskositäten von 10 - 100000, besonders bevorzugt von 50 - 1000 mm²/s.

Die Polysiloxane können zu einem geringen Grad verzweigt sein (beispielsweise < 2 Mol-% der Siloxaneinheiten), bzw. sind die Polymere aber substantiell linear, besonders bevorzugt vollständig linear. Zudem können die Substituenten R ihrerseits substituiert sein, beispielsweise mit N-haltigen Gruppen (beispielsweise Aminogruppen), Epoxygruppen, S-haltige Gruppen, Si-haltige Gruppen, O-haltige Gruppen usw.. Vorzugsweise sind mindestens 80% der Reste R Alkylreste, besonders bevorzugt Methylgruppen.
Das Organosiliconmaterial, das mit dem Polysiloxan in der Kettenverlängerungsreaktion reagiert, kann entweder ein zweites Polysiloxan sein, oder ein Molekül, das als Kettenverlängerer agiert. Wenn das Organosiliconmaterial ein Polysiloxan ist, hat es die vorstehend erwähnte generelle Struktur. In diesen Fällen besitzt ein Polysiloxan in der Reaktion (mindestens) eine reaktive Gruppe, und ein zweites Polysiloxan besitzt (mindestens) eine zweite reaktive Gruppe, die mit der ersten reagiert.
Falls das Organosiliconmaterial ein Kettenverlängerungs-Agens umfasst, kann dies ein Material sein wie beispielsweise ein Silan, ein Siloxan (beispielsweise Disiloxane oder Trisiloxan) oder ein Silazan. So kann beispielsweise eine Zusammensetzung, die ein Polysiloxan gemäß der vorstehend beschriebenen allgemeinen Struktur umfasst, welches mindestens eine Si-OH Gruppe aufweist, kettenverlängert werden, indem sie mit einem Alkoxysilan (beispielsweise einem Dialkoxysilan oder Trialkoxysilan) in Gegenwart von Zinn- oder Titan-haltigen Katalysatoren umgesetzt wird.
Die metallhaltigen Katalysatoren in der Kettenverlängerungsreaktion sind meist spezifisch für eine bestimmte Reaktion. Solche Katalysatoren sind im Stand der Technik bekannt und enthalten beispielsweise Metalle wie Platin, Rhodium, Zinn, Titan, Kupfer, Blei, etc.. In einer bevorzugten Kettenverlängerungsreaktion wird ein Polysiloxan mit mindestens einer aliphatisch ungesättigten Gruppe, vorzugsweise einer Endgruppe, mit einem Organosiliconmaterial in Gegenwart eines Hydrosilylierungskatalysators zur Reaktion gebracht, das ein Siloxan oder Polysiloxan mit mindestens einer (vorzugsweise endständigen) Si-H Gruppe ist. Das Polysiloxan besitzt mindestens eine aliphatisch ungesättigte Gruppe und genügt der allgemeinen oben angegeben Formel, in der R und n wie vorstehend definiert sind, wobei im Durchschnitt zwischen 1 und 2 Gruppen R eine aliphatisch ungesättigte Gruppe pro Polymer besitzen. Repräsentative aliphatisch ungesättigte Gruppen sind beispielsweise Vinyl, Allyl, Hexenyl und Cyclohexenyl oder eine Gruppe R²CH=CHR³, in der R² für eine divalente aliphatische an das Silicium gebundene Kette und R³ für ein Wasserstoffatom oder eine Alkylgruppe steht. Das Organosiliconmaterial mit mindestens einer Si-H Gruppe hat vorzugsweise die oben genannte Struktur, in der R und n wie vorstehend definiert sind und wobei im Durchschnitt zwischen 1 und 2 Gruppen R ein Wasserstoff bedeuten und n = 0 oder eine positive ganze Zahl ist.
Dieses Material kann ein Polymer oder ein niedermolekulares Material wie ein Siloxan sein (beispielsweise ein Disiloxane oder ein Trisiloxan).
Das Polysiloxan mit mindestens einer aliphatisch ungesättigten Gruppe und das Organosiliconmaterial mit mindestens einer Si-H Gruppe reagieren in Gegenwart eines Hydrosilylierungskatalysators. Solche Katalysatoren sind aus dem Stand der Technik bekannt und umfassen beispielsweise Platin- und Rhodium-enthaltende Materialien. Die Katalysatoren können jede bekannte Form annehmen, beispielsweise auf Trägermaterialien (wie beispielsweise Silica Gel oder Aktivkohle) aufgebrachtes Platin oder Rhodium oder andere geeignete Compounds wie Platinchlorid, Salze von Platin- oder Chloroplatinsäuren. Ein wegen der guten Dispergierbarkeit in Organosiliconsystemen und der geringen Farbveränderungen bevorzugter Katalysator ist Chloroplatinsäure entweder als kommerziell verfügbares Hexahydrat oder in wasserfreier Form.
Bei einer weiteren bevorzugten Kettenenrveiterungsreaktion wird ein Polysiloxan mit mindestens einer Si-OH Gruppe, vorzugsweise einer Endgruppe, mit einem Organosiliconmaterial zur Reaktion gebracht, das mindestens eine Alkoxygruppe besitzt, vorzugsweise ein Siloxan mit mindestens einer Si-OR Gruppe oder ein Alkoxysilan mit mindestens zwei Alkoxygruppen. Hierbei wird als Katalysator wieder ein metallhaltiger Katalysator eingesetzt.
Für die Reaktion einer Si-OH Gruppe mit einer Si-OR Gruppe existieren viele literaturbekannte Katalysatoren, beispielsweise Organometallverbindungen wie Organozinnsalze, Titanate oder Titanchelate bzw. -komplexe. Beispiele umfassen Zinn-octoat, Dibutylzinn-dilaurat, Dibutylzinn-diacetat, Dimethylzinn-dineodecanoat, Dibutylzinn-dimethoxid, Isobutylzinn-triceroat, Dimethylzinn-dibutyrat, Dimethylzinn-dineodecanoat, Triethylzinn-tartrat, Zinnoleat, Zinnnaphthenat, Zinnbutyrat, Zinnacetat, Zinnbenzoat, Zinnsebacat, Zinnsuccinat, Tetrabutyltitanat, Tetraisopropyltitanate, Tetraphenyltitanat, Tetraoctadecyltitanat, Titan-naphthanat, Ethyltriethanolamin-Titanat, Titan-diisopropyl-diethyl-acetoacetat, Titan-diisopropoxy-diacetyl-acetonat und Titan-tetra-Alkoxide, bei denen das Alkoxid Butoxy oder Propoxy ist.
Erfindungsgemäß ebenfalls bevorzugte kosmetische oder dermatologische Zubereitungen sind dadurch gekennzeichnet, dass sie mindestens ein Silicon der Formel IV

R₃Si-[O-SiR₂]ₓ-(CH₂)ₙ[O-SiR₂]_{y}O-SiR₃ (IV)

enthalten, in der R für gleiche oder verschiedene Reste aus der Gruppe -H, -Phenyl, -Benzyl, -CH₂-CH(CH₃)Ph, der C₁₋₂₀-Alkylreste, vorzugsweise -CH₃, -CH₂CH₃, -CH₂CH₂CH₃, -CH(CH₃)₂, -CH₂CH₂CH₂CH₃, -CH₂CH(CH₃)₂, -CH(CH₃)CH₂CH₃, -C(CH₃)₃, steht, x bzw. y für eine Zahl von 0 bis 200, vorzugsweise von 0 bis 10, weiter bevorzugt von 0 bis 7 und insbesondere 0, 1, 2, 3, 4, 5 oder 6, stehen, und n für eine Zahl von 0 bis 10, bevorzugt von 1 bis.8 und insbesondere für 2, 3, 4, 5, 6 steht.

Eine weitere bevorzugte Ausführungsform der Erfindung ist dadurch gekennzeichnet, dass das mindestens eine flüssige Siliconöl in einer Gesamtmenge von 1 - 40 Gew.-%, bevorzugt 5 - 30 Gew.-%, besonders bevorzugt 10 - 25 Gew.-%, außerordentlich bevorzugt 15 - 20 Gew.-%, jeweils bezogen auf die gesamte Zusammensetzung, enthalten ist.

Eine bevorzugte Ausführungsform der Erfindung ist dadurch gekennzeichnet, dass die ölige Phase II weiterhin mindestens eine weitere flüssige Ölkomponente, die kein Siliconöl und kein Parfüm ist, enthält.

Eine erfindungsgemäß besonders bevorzugte Gruppe weiterer einsetzbarer Ölkomponenten sind flüssige Paraffinöle und synthetische Kohlenwasserstoffe sowie Di-n-alkylether mit insgesamt 12 bis 36 C-Atomen, insbesondere 12 bis 24 C-Atomen, wie beispielsweise Di-n-octylether, Di-n-decylether, Din-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethylhexyl)-cyclohexan (Cetiol^{®} S) und Di-n-octylether (Cetiol^{®} OE) können besonders bevorzugt sein.

Erfindungsgemäß außerordentlich bevorzugte Ölkomponenten sind ausgewählt aus Fettsäure- und Fettalkoholestem. Besonders bevorzugt sind die Ester aus einem einwertigen geradkettigen oder verzweigten Alkohol mit 1 bis 17 Kohlenstoffatomen und einer geradkettigen oder verzweigten Fettsäure mit 3 bis 24 Kohlenstoffatomen. Außerordentlich bevorzugt sind die Produkte der Veresterung von Fettsäuren mit 8 bis 24 C-Atomen wie beispielsweise Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z. B. bei der Druckspaltung von natürlichen Fetten und Ölen, bei der Reduktion von Aldehyden aus der Roelen'schen Oxosynthese oder der Dimerisierung von ungesättigten Fettsäuren anfallen, mit Alkoholen wie beispielsweise Methanol, Ethanol, n-Propanol, Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z. B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß besonders bevorzugt sind Isopropylmyristat, Isononansäure-C16-18-alkylester (Cetiol^{®} SN), Stearinsäure-2-ethylhexylester (Cetiol^{®} 868), Cetyloleat und n-Butylstearat.
Eine weitere bevorzugte Ausführungsform der Erfindung ist dadurch gekennzeichnet, dass die ölige Phase II als weitere flüssige Ölkomponente mindestens einen Mono-, Di- oder Polyester aus einem geradkettigen oder verzweigten, ein- oder mehrwertigen Alkohol mit 1 bis 17 Kohlenstoffatomen und einer geradkettigen oder verzweigten Fettsäure mit 3 bis 24 Kohlenstoffatomen enthält.

Eine weitere bevorzugte Ausführungsform der Erfindung ist dadurch gekennzeichnet, dass die mindestens eine weitere flüssige Ölkomponente, die einen Mono-, Di- oder Polyester aus einem geradkettigen oder verzweigten, ein- oder mehrwertigen Alkohol mit 1 bis 17 Kohlenstoffatomen und einer geradkettigen oder verzweigten Fettsäure mit 3 bis 24 Kohlenstoffatomen darstellt, eine Molmasse von 200 - 1500 g/mol, bevorzugt 220 - 500 g/mol, besonders bevorzugt von 230 - 430 g/mol aufweist.

Weiterhin stellen auch Dicarbonsäureester wie Di-n-butyladipat, Di-(2-ethylhexyl)-adipat, Di-(2-ethylhexyl)-succinat und Di-isotridecylazelaat sowie Diolester wie Ethylenglykol-dioleat, Ethylenglykol-di-isotridecanoat, Propylenglykol-di(2-ethylhexanoat), Propylenglykol-di-isostearat, Propylenglykol-dipelargonat, Butandiol-di-isostearat und Neopentylglykoldi-capylat erfindungsgemäß bevorzugte Ölkomponenten dar, ebenso komplexe Ester wie z. B. das Diacetyl-glycerinmonostearat.

Eine weitere bevorzugte Ausführungsform der Erfindung ist dadurch gekennzeichnet, dass der Ester ausgewählt ist aus Diisopropyladipat, Di-n-butyladipat, 2-Ethylhexylhexanoat, Ethyllaurat, Methylmyristat, Isodecylneopentanoat, Ethylmyristat, Myristylpropionat, 2-Ethylhexyl-2-ethylhexanoat, 2-Ethylhexyloctanoat, 2-Ethylhexylcaprat/caprylat, Methylpalmitat, Butylmyristat, Isobutylmyristat, Ethylpalmitat, Isohexyllaurat, Hexyllaurat, Isodecylneopentanoat (242,4), Isotridecylneopentanoat (270,4), Iso-stearylneopentanoat (354,6), Octyldodecylneopentanoat (382,7), Isononylisononanoat (284,5), Octylisononanoat (270,4), Isodecylisononanoat (298,5), Isotridecylisononanoat (340,6), Isostearylisononanoat (410,7), Isopropylmyristat (270,5), Isopropylpalmitat (298,5), Isopropylstearat (326,6), Isopropylisostearat (326,6), Cetyloctanoat (368,6), Tridecyloctanoat (326,6), PEG-4-diheptanoat (418,5) und 2-Ethylhexylpalmitat (368,6), C₁₂-C₁₅-Alkylbenzoat, Neopentylglycoldiheptanoat (328,5), Propylenglycoldiethyl-2-hexanoat (328,5), 2-Ethylhexyl-2-ethylhexanoat (256,4), Glyceryltriheptanoat (428,6), oder Gemischen hiervon. Die Zahlen in Klammern geben die Molmasse in g/mol an.
Eine weitere bevorzugte Ausführungsform der Erfindung ist dadurch gekennzeichnet, dass die mindestens eine weitere flüssige Ölkomponente, die einen Mono-, Di- oder Polyester aus einem geradkettigen oder verzweigten, ein- oder mehrwertigen Alkohol mit 1 bis 17 Kohlenstoffatomen und einer geradkettigen oder verzweigten Fettsäure mit 3 bis 24 Kohlenstoffatomen darstellt, in einer Gesamtmenge von 0,1 - 5 Gew.-%, bevorzugt 0,5 - 3 Gew.-%, besonders bevorzugt 1 - 2 Gew.-%, jeweils bezogen auf die gesamte Zusammensetzung, enthalten ist.

Erfindungsgemäß einsetzbare Ölkomponenten sind schließlich auch Dialkylcarbonate, wie sie in der DE 19710154, auf die ausdrücklich Bezug genommen wird, ausführlich beschrieben werden. Dioctylcarbonate, insbesondere das Di-2-ethylhexylcarbonat, sind ebenfalls bevorzugte Ölkomponenten in Rahmen der vorliegenden Erfindung.

Eine weitere bevorzugte Ausführungsform der Erfindung ist dadurch gekennzeichnet, dass die ölige Phase II als weitere flüssige Ölkomponente mindestens einen unter Normalbedingungen flüssigen Glycerintriester von verzweigten oder linearen, gesättigten oder ungesättigten C₆ - C₂₂-Fettsäuren enthält. Bevorzugte flüssige Glycerintriester von verzweigten oder linearen, gesättigten oder ungesättigten C₆-C₂₂-Fettsäuren sind ausgewählt aus synthetischen Glycerintriestem von linearen C₆ -, C₈- und C₁₀-Fettsäuren, insbesondere Capric/Caprylic Triglycerides, z. B. die Handelsprodukte Myritol^{®} 318, Myritol^{®} 331 (Cognis) oder Miglyol^{®} 812 (Hüls), weiterhin Glyceryltriisostearin und die Handelsprodukte Estol^{®} GTEH 3609 (Uniqema) oder Myritol^{®} GTEH (Cognis) mit verzweigten Fettsäureresten. Weniger bevorzugt, aber im Einzelfall auch sehr gut geeignet können natürliche Öle, z.B. Sojaöl, Baumwollsaatöl, Sonnenblumenöl, Palmöl, Palmkernöl, Leinöl, Mandelöl, Rizinusöl, Maisöl, Olivenöl, Rapsöl, Sesamöl, Distelöl, Weizenkeimöl, Pfirsichkemöl und die flüssigen Anteile des Kokosöls und dergleichen sein. Natürliche Öle stammen von längeren Fettsäuren (C₁₄ und höher) ab; sie sind unter Normalbedingungen flüssig, da sie einen hohen Anteil an ungesättigten Fettsäuren aufweisen. Diese ungesättigten Fettsäureanteile sind oxidationsempfindlich und können zu einem schnelleren Produktverderb führen. Außerdem ist das fettende Hautgefühl der natürlichen Öle insbesondere für die Gesichtsreinigung und das Abschminken eher nachteilig. Daher sind erfindungsgemäß Glycerintriester bevorzugt, die nur von gesättigten Fettsäuren abstammen. Derartige Glycerintriester sind aber nur dann unter Normalbedingungen flüssig, wenn die Alkylkettenlänge der Fettsäuren nicht zu groß ist. Daher sind die Glycerintriester von linearen C₆ -, C₈- und C₁₀-Fettsäuren, insbesondere Capric/Caprylic Triglycerides, erfindungsgemäß besonders bevorzugt.

Eine weitere bevorzugte Ausführungsform der Erfindung ist dadurch gekennzeichnet, dass der mindestens eine flüssige Glycerintriester von verzweigten oder linearen, gesättigten oder ungesättigten C₆-C₂₂-Fettsäuren in einer Gesamtmenge von 0,1 - 5 Gew.-%, bevorzugt 0,5 - 3 Gew.-%, besonders bevorzugt 1 - 2 Gew.-%, jeweils bezogen auf die gesamte Zusammensetzung, enthalten ist.

Eine weitere bevorzugte Ausführungsform der Erfindung ist dadurch gekennzeichnet, dass die ölige Phase II mindestens ein Siliconöl, mindestens einen Ester, der aus einem geradkettigen oder verzweigten Alkohol mit 1 bis 17 Kohlenstoffatomen und einer geradkettigen oder verzweigten Fettsäure mit 3 bis 24 Kohlenstoffatomen hergestellt ist, und mindestens einen flüssigen Glycerintriester von verzweigten oder linearen, gesättigten oder ungesättigten C₆ - C₂₂-Fettsäuren enthält.

Die erfindungsgemäßen Zusammensetzungen enthalten mindestens einen Emulgator und oder mindestens ein Tensid. Besonders bevorzugt ist mindestens ein Emulgator oder Tensid mit einem HLB-Wert von 8 bis 18 und vorzugsweise von 10 bis 18 enthalten. Weiterhin ist es besonders bevorzugt - sofern ein Gemisch aus Emulgatoren und/oder Tensiden enthalten ist -, dass das gesamte Gemisch einen (mittleren) HLB-Wert von 8 bis 18 und vorzugsweise von 10 bis 18 aufweist.

Als Emulgatoren oder Tenside eignen sich bevorzugt
- Anlagerungsprodukte von 4 bis 150 Mol Ethylenoxid und/oder 0 bis 5 Mol Propylenoxid an lineare Fettalkohole mit 8 bis 22 C-Atomen oder an Fettsäuren mit 12 bis 22 C-Atomen in der Alkylgruppe,
- C₁₂-C₂₂-Fettsäuremono- und -diester von Anlagerungsprodukten von 1 bis 150 Mol Ethylenoxid an Polyole mit 3 bis 6 Kohlenstoffatomen, insbesondere an Glycerin,
- C₈-C₂₂-Alkylmono- und -oligoglycoside und deren ethoxylierte Analoga,
- Anlagerungsprodukte von 5 bis 150 Mol Ethylenoxid an Rizinusöl oder gehärtetes Rizinusöl,
- Partialester von Polyolen mit 3-6 Kohlenstoffatomen mit gesättigten Fettsäuren mit 8 bis 22 C-Atomen,
- Phospholipide. Hierunter werden vor allem die Glucose-Phospholipide, die z.B. als Lecithine bzw. Phosphatidylcholine aus z.B. Eidotter oder Pflanzensamen (z.B. Sojabohnen) gewonnen werden, verstanden.
- Fettsäureester von Zuckern und Zuckeralkoholen wie Sorbit, die gegebenenfalls mit 4 bis 150 Mol Ethylenoxid und/oder 0 bis 5 Mol Propylenoxid alkoxyliert sein können.

Bevorzugt können die erfindungsgemäßen Zusammensetzungen mindestens einen nichtionischen Emulgator mit einem HLB-Wert von 8 bis 18 enthalten. Nichtionische Emulgatoren mit einem HLB-Wert von 10 bis 18 können erfindungsgemäß besonders bevorzugt sein.

Erfindungsgemäße Zusammensetzungen sind dadurch gekennzeichnet, dass der gesamte Emulgator- und/oder Tensidgehalt 0,01 bis weniger als 0,1 Gew.-%, bevorzugt 0,05 - 0,06 Gew.-% beträgt, jeweils bezogen auf die gesamte Zusammensetzung.

Erfindungsgemäße Zusammensetzungen sind weiterhin dadurch gekennzeichnet, dass mindestens ein verdickendes Polymer enthalten ist.

Bevorzugte verdickende Polymere sind ausgewählt aus Polysacchariden und Polysaccharidderivaten, insbesondere den gewünschtenfalls chemisch modifizierten Cellulosen, Guar, Celluloseethern, Hydroxyalkylcellulosen wie Hydroxyethylcellulose, Hydroxypropylcellulose, Hydroxypropylmethylcellulose, Methylcellulose, Hydroxymethylcellulose, Carboxymethylcellulose, weiterhin insbesondere Stärken sowie Stärkeabbauprodukte wie Amylose und Amylopektin, chemisch und/oder thermisch modifizierte Stärken, z. B. Hydroxypropylstärkephosphat, Dihydroxypropyldistärkephosphat, Aluminiumstärkeoctenylsuccinat (beispielsweise aus der Handelsproduktserie Dry Flo^{®}), Natriumstärkeoctenylsuccinat, weiterhin Chitosan und dessen Derivaten. Weitere bevorzugte verdickende Polymere sind ausgewählt aus anionischen Polymeren, die Carboxylat- und/oder Sulfonatgruppen und als Monomere zum Beispiel Acrylsäure, Methacrylsäure, Crotonsäure, Maleinsäureanhydrid und 2-Acrylamido-2-methylpropansulfonsäure enthalten. Dabei können die sauren Gruppen ganz oder teilweise als Natrium-, Kalium-, Ammonium-, Mono- oder Triethanolammonium-Salz vorliegen. Bevorzugte Monomere sind 2-Acrylamido-2-methylpropansulfonsäure und Acrylsäure. Ganz besonders bevorzugte anionische Polymere enthalten als alleiniges Monomer oder als Comonomer 2-Acrylamido-2-methyl-propansulfonsäure, wobei die Sulfonsäuregruppe ganz oder teilweise in Salzform vorliegen kann. Innerhalb dieser Ausführungsform ist es bevorzugt, Copolymere aus mindestens einem anionischen Monomer und mindestens einem nichtionischen Monomer einzusetzen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Vinylpyrrolidon, Vinylether und Vinylester. Bevorzugte anionische Copolymere sind Acrylsäure-Acrylamid-Copolymere sowie insbesondere Polyacrylamidcopolymere mit Sulfonsäuregruppen-haltigen Monomeren. Ein besonders bevorzugtes anionisches Copolymer besteht aus 70 bis 55 Mol-% Acrylamid und 30 bis 45 Mol-% 2-Acrylamido-2-methylpropansulfonsäure, wobei die Sulfonsäuregruppen ganz oder teilweise als Natrium-, Kalium-, Ammonium-, Mono- oder Triethanolammonium-Salz vorliegen. Dieses Copolymer kann auch vernetzt vorliegen, wobei als Vernetzungsagentien bevorzugt polyolefinisch ungesättigte Verbindungen wie Tetraallyloxyethan, Allylsucrose, Allylpentaerythrit und Methylen-bisacrylamid zum Einsatz kommen. Ein solches Polymer ist in dem Handelsprodukt Sepigel^{®}305 der Firma SEPPIC enthalten. Die Verwendung dieses Compounds hat sich im Rahmen der erfindungsgemäßen Lehre als besonders vorteilhaft erwiesen. Auch die unter der Bezeichnung Simulgel^{®}600 als Compound mit Isohexadecan und Polysorbat-80 vertriebenen Natriumacryloyldimethyltaurat-Acrylamid-Copolymere haben sich als erfindungsgemäß besonders wirksam erwiesen. Auch die unter der Bezeichnung Simulgel^{®} NS als Compound mit Squalan und Polysorbat-60 vertriebenen Natriumacryfoyldimethyltaurat-Hydroxyethylacrylat-Copolymere haben sich als erfindungsgemäß besonders wirksam erwiesen. Auch die unter der Bezeichnung Simulgel^{®}EG als Compound mit Isohexadecan und Polysorbat-80 vertriebenen Natriumacryloyldimethyltaurat-Natriumacrylat-Copolymere haben sich als erfindungsgemäß besonders wirksam erwiesen. Auch die unter der Bezeichnung Simulgel^{®}EPG als Compound mit Polyisobuten und Caprylyl/Capryl Glucoside vertriebenen Natriumacryloyldimethyltaurat-Natriumacrylat-Copolymere haben sich als erfindungsgemäß besonders wirksam erwiesen. Auch die unter der Bezeichnung Sepiplus^{®} 400 als Compound mit Polyisobuten und Polysorbat-20 vertriebenen Acryloyldimethyltaurat-Copolymere mit der INCI-Bezeichnung Polyacrylate-13 haben sich als erfindungsgemäß besonders wirksam erwiesen.

Weitere besonders bevorzugte anionische Homopolymere sind unvernetzte und vernetzte Polyacrylsäuren. Dabei können Allylether von Pentaerythrit, von Sucrose und von Propylen bevorzugte Vernetzungsagentien sein. Solche Verbindungen sind zum Beispiel die Handelsprodukte Carbopol^{®}, insbesondere Carbopol^{®} 954 und 980. Ein besonders bevorzugtes anionisches Copolymer enthält als Monomer zu 80 - 98 % eine ungesättigte C₃₋₆-Carbonsäure oder ihr Anhydrid sowie zu 2 - 20 % Acrylsäureester von gesättigten C₁₀₋₃₀-Carbonsäuren, wobei das Copolymer mit den vorgenannten Vernetzungsagentien vernetzt sein kann. Entsprechende Handelsprodukte sind beispielsweise Pemulen^{®} und die Carbopol^{®}-Typen 1342, 1382, Ultrez 21 und ETD 2020 (ex B.F. Goodrich, Noveon, Lubrizol Advanced Materials).

Besonders bevorzugte verdickende Polymere sind weiterhin ausgewählt aus Polyvinylalkoholen, Polyvinylpyrrolidon, Polyacrylamiden, Polyvinylacetaten und Polyalkylenglycolen mit Molekulargewichten von mehr als 1000 D. Auch wasserlösliche Copolymerisate der Acrylsäure, des Acrylamids, des Vinylpyrrolidons und anderer wasserlöslicher Monomeren mit nicht-wasserlöslichen Comonomeren sind als verdickende Polymere im Sinne der vorliegenden Erfindung bevorzugt.
Außerordentlich bevorzugt sind die verdickende Polymere ausgewählt aus Polyethylenglykolen, Polypropylenglykolen, EO-PO-Polyalkylenglykolen und Polyvinylpyrrolidon, jeweils mit einem mittleren Molekulargewicht von mindestens 1000 D und besonders bevorzugt von 5000 bis 50000 D, wobei sich die genannten Werte auf mittels der Gelpermeation bestimmte Molekulargewichtswerte beziehen.

Ebenfalls außerordentlich bevorzugt sind die verdickende Polymere ausgewählt aus nichtionischen Polymeren.

Das mindestens eine verdickende Polymer ist in einer Gesamtmenge von 0,1 - 2 Gew.-%, bevorzugt 0,3 - 1 Gew.-% und besonders bevorzugt 0,3 - 0,5 Gew.-%, jeweils bezogen auf die gesamte Zusammensetzung, enthalten.

Weiterhin ist es erfindungsgemäß bevorzugt, wenn das Gewichtsverhältnis von verdickendem Polymer und Emulgator 1 bis 30, vorzugsweise 4 bis 10, besonders bevorzugt 5 - 8, beträgt. Sofern mehrere wasserlösliche Polymere und/oder mehrere Emulgatoren in der Zusammensetzung enthalten sind, gelten diese Werte jeweils für das Verhältnis zwischen der Gesamtheit der wasserlöslichen Polymeren und Emulgatoren.

Der Wassergehalt beträgt erfindungsgemäß 50 - 85 Gew.-%, bevorzugt 60 - 75 Gew.-% und besonders bevorzugt 65 - 70 Gew.-%, jeweils bezogen auf die gesamte Zusammensetzung.

Weiterhin kann es bevorzugt sein, die einzelnen Phasen/Schichten mit Farbstoffen anzufärben, um ein besonders gutes optisches Erscheinungsbild des Mittels zu erzielen. Diese Farbstoffe sind bevorzugt nur in der wässrigen oder nur in mindestens einer nichtwässrigen Phase in einer Menge löslich, die eine entsprechende Einfärbung für den Betrachter sichtbar erscheinen lässt. Es ist auch möglich, sowohl die nichtwässrige als auch die wässrige Phase mit verschiedenen Farbstoffen, bevorzugt in verschiedenen Farben, einzufärben. Das alleinige Anfärben einer nichtwässrigen Phase ist jedoch bevorzugt.

In einer weiteren bevorzugten Ausführungsform enthalten die erfindungsgemäßen Zusammensetzungen mindestens ein wasserlösliches Polyol, ausgewählt aus wasserlöslichen Diolen, Triolen und höherwertigen Alkoholen sowie Polyethylenglycolen. Derartige Wirkstoffe können die Stabilitätseigenschaften der erfindungsgemäßen Zusammensetzungen in überraschender Weise positiv beeinflussen. Unter den Diolen eignen sich bevorzugt C₂-C₁₂-Diole, insbesondere 1,2-Propylengiycol, Butylenglycole wie z. B. 1,2-Butylenglycol, 1,3-Butylenglycol und 1,4-Butylenglycol, Pentandiole, z. B. 1,2-Pentandiol, sowie Hexandiole, z. B. 1,6-Hexandiol und 1,2-Hexandiol sowie 1,2-Octandiol. Weiterhin bevorzugt geeignet sind Glycerin und technische Oligoglyceringemische mit einem Eigenkondensationsgrad von 1,5 bis 10 wie etwa technische Diglyceringemische mit einem Diglyceringehalt von 40 bis 50 Gew.-% oder Triglycerin, weiterhin 1,2,6-Hexantriol sowie Polyethylenglycole (PEG), die nur aus Ethylenoxideinheiten bestehen, mit einem durchschnittlichen Molekulargewicht von 100 bis 1.000 Dalton, beispielsweise PEG-400, PEG-600 oder PEG-1000. Weitere geeignete höherwertige Alkohole sind die C₄-, C₅- und C₆-Monosaccharide und die entsprechenden Zuckeralkohole, z. B. Mannit oder Sorbit. Unter "wasserlöstich" wird erfindungsgemäß verstanden, dass sich mindestens 5 g des Polyols in 100 g Wasser bei 20°C lösen.

Die erfindungsgemäßen Zusammensetzungen enthalten das mindestens eine wasserlösliche Polyol bevorzugt in Gesamtmengen von 1 - 50 Gew.-%, besonders bevorzugt 4 - 15 Gew.-% und außerordentlich bevorzugt 6 - 10 Gew.-%, jeweils bezogen auf die gesamte Zusammensetzung.

Eine weitere bevorzugte Ausführungsform der Erfindung ist dadurch gekennzeichnet, dass die erfindungsgemäßen Zusammensetzungen mindestens ein Salz, ausgewählt aus Trikaliumphoaphat, Dikaliumhydrogenphosphat, Kaliumdihydrogenphosphat, Natriumchlorid und Kaliumchlorid, enthalten. Die erfindungsgemäßen Zusammensetzungen enthalten mindestens eines dieser Salze bevorzugt in einer Gesamtmenge von 0,1 - 5 Gew.-%, besonders bevorzugt 0,2 - 3 Gew.-% und außerordentlich bevorzugt 1 - 2 Gew.-%, jeweils bezogen auf die gesamte Zusammensetzung.
Eine weitere bevorzugte Ausführungsform der Erfindung ist dadurch gekennzeichnet, dass die erfindungsgemäßen Zusammensetzungen mindestens eine Duftstoffkomponente enthalten ist.

Als Duftstoffkomponente können Parfüme, Parfümöle oder Parfümölbestandteile eingesetzt werden. Parfümöle bzw. Duftstoffe können erfindungsgemäß einzelne Riechstoffverbindungen, z. B. die synthetischen Produkte vom Typ der Ester, Ether, Aldehyde, Keton, Alkohole und Kohlenwasserstoffe sein. Riechstoffverbindungen vom Typ der Ester sind z.B. Benzylacetat, Phenoxyethylisobutyrat, ptert.-Butylcyclohexylacetat, Linalylacetat, Dimethylbenzylcarbinylaretat (DMBCA), Phenylethylacetat, Benzylacetat, Ethylmethylphenylglycinat, Allylcyclohexylpropionat, Styrallylpropionat, Benzylsalicylat, Cyclohexylsalicylat, Floramat, Melusat und Jasmecyclat. Zu den Ethern zählen beispielsweise Benzylethylether und Ambroxan , zu den Aldehyden z.B. die linearen Alkanale mit 8 - 18 C-Atomen, Citral, Citronellal, Citronellyloxy-acetaldehyd, Cyclamenaldehyd, Lilial und Bourgeonal, zu den Ketonen z.B. die Jonone, alpha-Isomethylionon und Mthylcedrylketon, zu den Alkoholen Anethol, Citronellol, Eugenol, Geraniol, Linalool, Phenylethylalkohol und Terpineol, zu den Kohlenwasserstoffen gehören hauptsächlich die Terpene wie Limonen und Pinen. Bevorzugt werden jedoch Mischungen verschiedener Riechstoffe verwendet, die gemeinsam eine ansprechende Duftnote erzeugen.

In einer weiteren bevorzugten Ausführungsform enthalten die erfindungsgemäßen Zusammensetzungen mindestens einen sebumregulierenden Wirkstoff. Erfindungsgemäß bevorzugte sebumregulierende Wirkstoffe sind ausgewählt aus 10-Hydroxydecansäure, Sebacinsäure, Azelainsäure und den Estern der Azelainsäure, insbesondere Kaliumazeloyldiglycinat, 1,10-Decandiol und mindestens einem Extrakt aus Spiraea Ulmaria sowie Mischungen der vorgenannten Substanzen. Bevorzugte Mischungen sind beispielsweise erhältlich als Handelsprodukt Acnacidol PG (Propylene Glycol, 10-Hydroxydecanoic acid, Sebacic acid, 1,10-Decandiol) von Vincience erhältlich. Ein bevorzugter Extrakt aus Spiraea Ulmaria ist z. B. im Produkt Seboregul 2 der Firma Silab enthalten. Kaliumazeloyldiglycinat ist z. B. in dem Produkt Azeloglicina der Firma Sinerga enthalten. Erfindungsgemäß besonders bevorzugte kosmetische oder dermatologische Zusammensetzungen sind dadurch gekennzeichnet, dass sie mindestens einen sebumregulierenden Wirkstoff in Gesamtmengen von 0,00001 bis 10 Gew.-%, bevorzugt 0,01 bis 5 Gew.-% und besonders bevorzugt 0,1 bis 1 -2 Gew.-%, jeweils bezogen auf Aktivsubstanz in der gesamten erfindungsgemäßen Zusammensetzung, enthalten.

In einer weiteren bevorzugten Ausführungsform enthalten die erfindungsgemäßen Zusammensetzungen mindestens einen Desoxyzucker oder ein mindestens einen Desoxyzucker-Baustein enthaltendes Polysaccharid.
Erfindungsgemäß bevorzugte Desoxyzucker sind ausgewählt aus Rhamnose und Fucose. Ein erfindungsgemäß besonders bevorzugtes, Desoxyzucker-Bausteine enthaltendes Polysaccharid ist das Handelsprodukt Fucogel^{®} von Solabia mit der INCI-Bezeichnung Biosaccharide Gum-1. Ein weiteres erfindungsgemäß besonders bevorzugtes. Desoxyzucker-Bausteine enthaltendes Polysaccharid ist das Handelsprodukt Rhamnosoft^{®} von Solabia mit der INCI-Bezeichnung Biosaccharide Gum-2. Ein erfindungsgemäß besonders bevorzugtes, Desoxyzucker-Bausteine enthaltendes Polysaccharid ist das Handelsprodukt Fucogenol^{®} von Solabia mit der INCI-Bezeichnung Biosaccharide Gum-3. Ein erfindungsgemäß besonders bevorzugtes, Desoxyzucker-Bausteine enthaltendes Polysaccharid ist das Handelsprodukt Glycofilm^{®} von Solabia mit der INCI-Bezeichnung Biosaccharide Gum-4. Erfindungsgemäß bevorzugt sind weiterhin Mischungen der vorgenannten, mindestens einen Desoxyzucker-Baustein enthaltenden Polysaccharide, beispielsweise die Mischung aus Biosaccharide Gum-2 und Biosaccharide Gum-3, erhältlich als Handelsprodukt Elastinol plus^{®} von Solabia. Erfindungsgemäß besonders bevorzugte kosmetische oder dermatologische Zusammensetzungen sind dadurch gekennzeichnet, dass sie mindestens einen Desoxyzucker und/ oder mindestens ein Desoxyzucker-Bausteine enthaltendes Polysaccharid in Gesamtmengen von 0,001 bis 5 Gew.-%, bevorzugt 0,01 bis 2 Gew.-% und besonders bevorzugt 0,1 bis 1 Gew.-%, jeweils bezogen auf die gesamte erfindungsgemäße Zusammensetzung, enthalten.

Ein weiterer Gegenstand der vorliegenden Anmeldung ist die nicht-therapeutische kosmetische Verwendung einer Zusammensetzung nach einem der Ansprüche 1 - 14 zur Pflege, zum Reinigen und/oder Abschminken der Haut, der Schleimhaut, der Augen und/oder der Haare.

Ein weiterer Gegenstand der vorliegenden Anmeldung ist ein nicht-therapeutisches, kosmetisches Verfahren zur Pflege, zum Reinigen und/oder Abschminken der Haut, der Schleimhaut, der Augen und/oder der Haare, dadurch gekennzeichnet, dass eine Zusammensetzung nach einem der Ansprüche 1 - 14 auf die Haut, Schleimhaut, Augen und/oder Haare aufgetragen wird.

Ein erfindungsgemäß bevorzugtes Verfahren zum Reinigen und/oder Abschminken der Haut, der Schleimhaut, der Augen und/oder der Haare ist dadurch gekennzeichnet, dass eine erfindungsgemäße Zusammensetzung nach einem der Ansprüche 1 - 14 mit einem Träger, Tuch, Pad, Bausch, Lappen, Schwamm oder Netzball auf die Haut, Schleimhaut, Augen und/oder Haare aufgetragen wird.

Weiterhin ist es bevorzugt, dass die erfindungsgemäße Zusammensetzung nach einem der Ansprüche 1 - 14 nach der Applikation auf die Haut, Schleimhaut, Augen und/oder Haare abgespült wird.

Weiterhin ist es bevorzugt, dass die erfindungsgemäße Zusammensetzung in einem transparenten Behälter verpackt ist.
Weiterhin ist es bevorzugt, dass die erfindungsgemäße Zusammensetzung in einem Sprühspender verpackt ist.
Weiterhin ist es bevorzugt, dass die erfindungsgemäße Zusammensetzung in einem transparenten Sprühspender verpackt ist.
Weiterhin ist es bevorzugt, dass die erfindungsgemäße Zusammensetzung verpackt ist und einen Anweisung an den Verbraucher umfasst, dass die Zusammensetzung vor dem Gebrauch zu schütteln ist.

Die folgenden Beispiele sollen die Erfindung näher erläutern, ohne sie hierauf zu beschränken.

### Ausführungsbeispiele

Soweit nicht anders vermerkt, sind alle Angaben Gewichtsteile.

### 1. Gesichtsreinigungsmittel und Make-up-Entferner

| | |
|---|---|
| CYCLOMETHICONE | 25.000000 |
| ISOPROPYL PALMITATE | 2.000000 |
| GLYCERIN, 86 %ig | 3.000000 |
| CAPRYLIC/CAPRIC TRIGLYCERIDE | 1.000000 |
| SODIUM CHLORIDE | 1.000000 |
| PHENOXYETHANOL | 0.500000 |
| BENZYL ALCOHOL | 0.500000 |
| PANTHENOL | 0.500000 |
| HYDROXYPROPYL METHYLCELLULOSE | 0.345000 |
| TOCOPHERYL ACETATE | 0.200000 |
| DIPOTASSIUM PHOSPHATE | 0.175000 |
| DISODIUM EDTA | 0.100000 |
| POTASSIUM PHOSPHATE | 0.100000 |
| MALIC ACID | 0.100000 |
| PEG-40 HYDROGENATED CASTOR OIL | 0.072000 |
| BUTYLENE GLYCOL | 0.355000 |
| BENZOPHENONE-4 | 0.050000 |
| ETHYLPARABEN | 0.000280 |
| METHYLPARABEN | 0.000280 |
| PROPYLPARABEN | 0.000085 |
| CI 16035 | 0.000085 |
| AQUA | ad 100.000000 |

Das zweischichtige Mittel nach Beispiel 1 wurde vor der Anwendung geschüttelt, bis eine optisch homogene milchige Emulsion entstand. Anschließend wurde eine zur Reinigung ausreichende Menge mit der Hand auf die Gesichtshaut gegeben, einmassiert und mit warmem Wasser abgespült.

Das Mittel nach Beispiel 1 wurde vor der Anwendung geschüttelt, bis eine optisch homogene milchige Emulsion entstand. Anschließend wurde eine zur Reinigung ausreichende Menge auf ein Wattepad oder ein nonwoven-Vlies aufgetragen. Mit dem Imprägnierten Träger wurden die Gesichtshaut, Lippen und die Augen abgeschminkt. In einem Verfahren wurde nicht nachgespült, in einem zweiten Verfahren wurde mit warmem Wasser nachgespült.

In jedem Fall wurde ein sehr guter Reinigungseffekt erzielt. Das Hautgefühl nach der Anwendung war sehr angenehm. Beim Abschminken der Augen trat kein Brennen oder Tränen der Augen auf.

### 2. Gesichtsreinigungsmittel und Make-up-Entferner

| | |
|---|---|
| CYCLOMETHICONE | 30.000000 |
| HEXYL LAURATE | 3.000000 |
| GLYCERIN, 86 %ig | 4.000000 |
| CAPRYLIC/CAPRIC TRIGLYCERIDE | 1.000000 |
| SODIUM CHLORIDE | 0.800000 |
| PHENOXYETHANOL | 0.500000 |
| MATRIXYL 3000 | 0.500000 |
| PANTHENOL | 0.500000 |
| HYDROXYPROPYL METHYLCELLULOSE | 0.400000 |
| MAGNESIUM ASCORBYL PHOSPHATE | 0.600000 |
| DIPOTASSIUM PHOSPHATE | 0.200000 |
| DISODIUM EDTA | 0.100000 |
| POTASSIUM PHOSPHATE | 0.150000 |
| SEBACIC ACID | 0.100000 |
| PEG-40 HYDROGENATED CASTOR OIL | 0.063000 |
| METHYLPARABEN | 0.000280 |
| PROPYLPARABEN | 0.000085 |
| CI 16035 | 0.000085 |
| AQUA | ad 100.000000 |

## Patentansprüche

1. Wässrige, mindestens ein Öl und mindestens einen Emulgator oder mindestens ein Tensid enthaltende, flüssige, mindestens zweischichtige Zusammensetzung, die mindestens eine kontinuierliche wässrige Phase I und mindestens eine diskontinuierliche ölige Phase II aufweist, wobei im Ruhezustand die ölige Phase II zusammen mit einem Teil einer wässrigen Phase I eine Emulsionsschicht, und ein weiterer Teil der wässrigen Phase I eine wässrige Schicht der Zusammensetzung bildet, und die Zusammensetzung sich durch mechanische Bewegung reversibel temporär in eine Emulsion überführen lässt, **dadurch gekennzeichnet, dass**
a) die ölige Phase II mindestens ein bei Normalbedingungen flüssiges Siliconöl enthält,
b) der Wassergehalt 50 - 85 Gew.-%, bevorzugt 60 - 75 Gew.-% und besonders bevorzugt 65 - 70 Gew.-%, jeweils bezogen auf die gesamte Zusammensetzung, beträgt,
c) der gesamte Emulgator- und/oder Tensidgehalt 0,01 bis weniger als 0,1 Gew.-% beträgt, jeweils bezogen auf die gesamte Zusammensetzung,
d) mindestens ein verdickendes Polymer In einer Gesamtmenge von 0,1 - 2 Gew.-%, bevorzugt 0,3 - 1 Gew.-% und besonders bevorzugt 0,3 - 0,5 Gew.-%, jeweils bezogen auf die gesamte Zusammensetzung, enthalten ist.

2. Zusammensetzung nach Anspruch 1, **dadurch gekennzeichnet, dass** das mindestens eine flüssige Siliconöl ausgewählt ist aus Siliconen der Formel (CH₃)₃Si-[O-Si(CH₃)₂]ₓ-O-Si(CH₃)₃, in der x für eine Zahl von 0 bis 50, vorzugsweise von 1 bis 20 und insbesondere 3 bis 10, steht, und/oder Siliconen der Formel 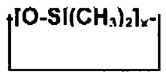 in der x für eine der Zahlen 3, 4, 5 oder 6 steht.

3. Zusammensetzung nach einem der vorhergehenden Ansprüche, **dadurch gekennzeichnet, dass** das mindestens eine flüssige Siliconöl in einer Gesamtmenge von 1 - 40 Gew.-%. bevorzugt 5 - 30 Gew.-%, besonders bevorzugt 10 - 25 Gew.-%, außerordentlich bevorzugt 15 - 20 Gew.-%, jeweils bezogen auf die gesamte Zusammensetzung, enthalten ist.

4. Zusammensetzung nach einem der vorhergehenden Ansprüche, **dadurch gekennzeichnet, dass** die ölige Phase II weiterhin mindestens eine weitere flüssige Ölkomponente, die kein Siliconöl und kein Duftstoff ist, enthält.

5. Zusammensetzung nach einem der vorhergehenden Ansprüche, **dadurch gekennzeichnet, dass** die ölige Phase II als weitere flüssige Ölkomponente mindestens einen Mono-, Di- oder Polyester aus einem geradkettigen oder verzweigten, ein- oder mehrwertigen Alkohol mit 1 bis 17 Kohlenstoffatomen und einer geradkettigen oder verzweigten Fettsäure mit 3 bis 24 Kohlenstoffatomen enthält.

6. Zusammensetzung nach Anspruch 5, **dadurch gekennzeichnet, dass** der Ester ausgewählt ist aus Diisopropyladipat, Di-n-butyladipat, 2-Ethylhexylhexanoat, Ethyllaurat, Methylmyristat, Isodecylneopentanoat, Ethylmyristat, Myristylpropionat, 2-Ethylhexyl-2-ethylhexanoat, 2-Ethylhexyloctanoat, 2-Ethylhexylcaprat/caprylat, Methylpalmitat, Butylmyristat, Isobutylmyristat, Ethylpalmitat, Isohexyllaurat, Hexyllaurat, Isodecylneopentanoat, Isotridecylneopentanoat, Isostearylneopentanoat, Octyldodecylneopentanoat, Isononylisononanoat, Octylisononanoat, Isodecylisononanoat, Isotridecylisononanoat, Isostearylisononanoat, Isopropylmyristat, Isopropylpalmitat, Isopropylstearat, Isopropylisostearat, Cetyloctanoat, Tridecyloctanoat, PEG-4-diheptanoat und 2-Ethylhexylpalmitat, C₁₂-C₁₅-Alkylbenzoat, Neopentylglycoldiheptanoat, Propylenglycoldiethyl-2-hexanoat, 2-Ethylhexyl-2-ethylhexanoat, Glyceryltriheptanoat oder deren Gemischen.

7. Zusammensetzung nach einem der Ansprüche 5 - 6, **dadurch gekennzeichnet, dass** die mindestens eine weitere flüssige Ölkomponente, die einen Mono-, Di- oder Polyester aus einem geradkettigen oder verzweigten, ein- oder mehrwertigen Alkohol mit 1 bis 17 Kohlenstoffatomen und einer geradkettigen oder verzweigten Fettsäure mit 3 bis 24 Kohlenstoffatomen darstellt, in einer Gesamtmenge von 0,1 - 5 Gew.-%, bevorzugt 0,5 - 3 Gew.-%, besonders bevorzugt 1 - 2 Gew.%, jeweils bezogen auf die gesamte Zusammensetzung, enthalten ist.

8. Zusammensetzung nach einem der vorhergehenden Ansprüche, **dadurch gekennzeichnet, dass** die ölige Phase II als weitere flüssige Ölkomponente mindestens einen unter Normalbedingungen flüssigen Glycerintriester von verzweigten oder linearen, gesättigten oder ungesättigten C₈ - C₂₂-Fettsäuren enthält.

9. Zusammensetzung nach Anspruch 8, **dadurch gekennzeichnet, dass** der mindestens eine flüssige Glycerintriester von verzweigten oder linearen, gesättigten oder ungesättigten C₆ - C₂₂-Fettsäuren in einer Gesamtmenge von 0,1 - 5 Gew.-%, bevorzugt 0,5 - 3 Gew.-%, besonders bevorzugt 1 - 2 Gew.-%, jeweils bezogen auf die gesamte Zusammensetzung, enthalten ist.

10. Zusammensetzung nach einem der vorhergehenden Ansprüche, **dadurch gekennzeichnet, dass** die ölige Phase II mindestens ein Siliconöl, mindestens einen Ester, der aus einem geradkettigen oder verzweigten Alkohol mit 1 bis 17 Kohlenstoffatomen und einer geradkettigen oder verzweigten Fettsäure mit 3 bis 24 Kohlenstoffatomen hergestellt ist, und mindestens einen flüssigen Glycerintriester von verzweigten oder linearen, gesättigten oder ungesättigten C₈ - C₂₂-Fettsäuren enthält.

11. Zusammensetzung nach einem der vorhergehenden Ansprüche, **dadurch gekennzeichnet, dass** die Emulgatoren und/oder Tenside ausgewählt sind aus
- Anlagerungsprodukten von 4 bis 150 Mol Ethylenoxid und/oder 0 bis 5 Mol Propylenoxid an lineare Fettalkohole mit 8 bis 22 C-Atomen oder an Fettsäuren mit 12 bis 22 C-Atomen in der Alkylgruppe,
- C₁₂-C₂₂-Fettsäuremono- und -diestern von Anlagerungsprodukten von 1 bis 150 Mol Ethylenoxid an Polyole mit 3 bis 6 Kohlenstoffatomen, insbesondere an Glycerin,
- C₈-C₂₂-Alkylmono- und -oligoglycosiden und deren ethoxylierte Analoga,
- Anlagerungsprodukten von 5 bis 150 Mol Ethylenoxid an Rizinusöl oder gehärtetes Rizinusöl,
- Partialestern von Polyolen mit 3-6 Kohlenstoffatomen mit gesättigten Fettsäuren mit 8 bis 22 C-Atomen,
- Fettsäureestern von Zuckern und Zuckeralkoholen wie Sorbit, die gegebenenfalls mit 4 bis 150 Mol Ethylenoxid und/oder 0 bis 5 Mol Propylenoxid alkoxyliert sein können,
- Phospholipiden.

12. Zusammensetzung nach einem der vorhergehenden Ansprüche, **dadurch gekennzeichnet, dass** das mindestens eine verdickende Polymer ausgewählt ist aus Polyethylenglycolen, Polypropylenglycolen, EO-PO-Polyalkylenglykolen und Polyvinylpyrrolidon, jeweils mit einem mittleren Molekulargewicht von mindestens 1000 D und besonders bevorzugt von 5000 bis 50000 D, Stärke, Guar, Celluloseethern, Hydroxyalkylcellulosen wie Hydroxyethylcellulose, Hydroxypropylcellulose, Hydroxypropylmethylcellulose, Methylcellulose, Hydroxymethylcellulose und Carboxymethylcellulose.

13. Zusammensetzung nach einem der vorhergehenden Ansprüche, **dadurch gekennzeichnet, dass** mindestens ein wasserlösliches Polyol, ausgewählt aus wasserlöslichen Diolen, Triolen und höherwertigen Alkoholen sowie Polyethylenglycolen, die nur aus Ethylenoxid-Einheiten bestehen, enthalten ist.

14. Zusammensetzung nach einem der vorhergehenden Ansprüche, **dadurch gekennzeichnet, dass** mindestens ein Salz, ausgewählt aus Trikaliumphosphat, Dikaliumhydrogenphosphat, Kaliumdihydrogenphosphat, Natriumchlorid und Kaliumchlorid, in einer Gesamtmenge von 0,1 - 5 Gew.-%, besonders bevorzugt 0,2 - 3 Gew.-% und außerordentlich bevorzugt 1 - 2 Gew.-%, jeweils bezogen auf die gesamte Zusammensetzung, enthalten ist.

15. Nicht-therapeutische, kosmetische Verwendung einer Zusammensetzung nach einem der Ansprüche 1 - 14 zur Pflege, zum Reinigen und/oder Abschminken der Haut, der Schleimhaut, der Augen und/oder der Haare.

16. Nicht-therapeutisches, kosmetisches Verfahren zur Pflege, zum Reinigen und/oder Abschminken der Haut, der Schleimhaut, der Augen und/oder der Haare, **dadurch gekennzeichnet, dass** eine Zusammensetzung nach einem der Ansprüche 1 - 14 auf Haut, Schleimhaut, Augen und/oder Haare aufgetragen wird.

17. Verfahren nach Anspruch 16, **dadurch gekennzeichnet, dass** eine Zusammensetzung nach einem der Ansprüche 1 - 14 mit einem Träger, Tuch, Pad, Bausch, Lappen, Schwamm oder Netzball auf die Haut aufgetragen wird.

18. Verfahren nach Anspruch 16 oder 17, **dadurch gekennzeichnet, dass** die Zusammensetzung nach einem der Ansprüche 1 - 14 nach der Applikation abgespült und/oder abgewischt wird.

## Claims

1. Aqueous, liquid, at least two-phase composition that comprises at least one oil and at least one emulsifier or at least one surfactant, and that has at least one continuous aqueous phase I and at least one discontinuous oily phase II, wherein in the quiescent state the oily phase II together with a part of an aqueous phase I forms an emulsion layer, and another part of the aqueous phase I forms an aqueous layer of the composition, and the composition can be temporarily reversibly converted by mechanical movement into an emulsion, **characterised in that**
a) the oily phase II comprises at least one silicone oil that is liquid under normal conditions,
b) the water content is 50 - 85 wt %, preferably 60 - 75 wt % and particularly preferably 65 - 70 wt %, in each case based on the total composition,
c) the total emulsifier content and/or surfactant content is 0.01 to less than 0.1 wt %, each based on the total composition,
d) at least one thickening polymer is comprised in a total amount of 0.1 - 2 wt %, preferably 0.3 - 1 wt % and particularly preferably 0.3 - 0.5 wt %, each based on the total composition.

2. Composition according to claim 1, **characterised in that** the at least one liquid silicone oil is selected from silicones of the Formula (CH₃)₃Si-[O-Si(CH₃)₂]ₓ-O-Si(CH₃)₃, in which x stands for a number from 0 to 50, preferably from 1 to 20 and especially 3 to 10, and/or from silicones of the Formula 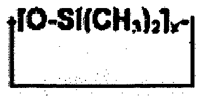 in which x stands for one of the numbers 3, 4, 5 or 6.

3. Composition according to one of the preceding claims, **characterised in that** the at least one liquid silicone oil is comprised in a total amount of 1 - 40 wt %, preferably 5 - 30 wt %, particularly preferably 10 - 25 wt %, exceptionally preferably 15 - 20 wt %, each relative to the total composition.

4. Composition according to one of the preceding claims, **characterised in that** the oily phase II further comprises at least one additional liquid oil component that is neither a silicone oil nor a fragrance.

5. Composition according to one of the preceding claims, **characterised in that** the oily phase II comprises as the additional liquid oil component at least one mono, di or polyester of a straight chain or branched monohydric or polyhydric alcohol containing 1 to 17 carbon atoms and a straight chain or branched fatty acid containing 3 to 24 carbon atoms.

6. Composition according to claim 5, **characterised in that** the ester is selected from diisopropyl adipate, di-*n*-butyl adipate, 2-ethylhexyl hexanoate, ethyl laurate, methyl myristate, *iso*decyl *neo*pentanoate, ethyl myristate, myristyl propionate, 2-ethylhexyl 2-ethylhexanoate, 2-ethylhexyl octanoate, 2-ethylhexyl caproate/caprylate, methyl palmitate, butyl myristate, *iso*butyl myristate, ethyl palmitate, *iso*hexyl laurate, hexyl laurate, isodecyl *neo*pentanoate, *iso*tridecyl *neo*pentanoate, *iso*stearyl *neo*pentanoate, octyldodecyl *neo*pentanoate, *iso*nonyl *iso*nonanoate, octyl *iso*nonanoate, *iso*decyl i*so*nonanoate, *iso*tridecyl *iso*nonanoate, *iso*stearyl *iso*nonanoate, isopropyl myristate, isopropyl palmitate, isopropyl stearate, isopropyl *iso*stearate, cetyl octanoate, tridecyl octanoate, PEG-4-diheptanoate and 2-ethylhexyl palmitate, C₁₂-C₁₅ alkyl benzoate, *neo*pentyl glycol diheptanoate, propylene glycol diethyl-2-hexanoate, 2-ethylhexyl 2-ethylhexanoate, glyceryl triheptanoate or their mixtures.

7. Composition according to one of claims 5 - 6, **characterised in that** the at least one additional liquid oil component that represents a mono, di or polyester of a straight chain or branched monohydric or polyhydric alcohol containing 1 to 17 carbon atoms and a straight chain or branched fatty acid containing 3 to 24 carbon atoms, is comprised in a total amount of 0.1 - 5 wt %, preferably 0.5 - 3 wt %, particularly preferably 1 - 2 wt %, each based on the total composition.

8. Composition according to one of the preceding claims, **characterised in that** the oily phase II comprises as the additional liquid oil component at least one glycerine triester of branched or linear, saturated or unsaturated C₆ - C₂₂ fatty acids, said triester being liquid under normal conditions.

9. Composition according to claim 8, **characterised in that** the at least one liquid glycerine triester of branched or linear, saturated or unsaturated C₆ - C₂₂ fatty acids is comprised in a total amount of 0.1 - 5 wt %, preferably 0.5 - 3 wt %, particularly preferably 1 - 2 wt %, each relative to the total composition.

10. Composition according to one of the preceding claims, **characterised in that** the oily phase II comprises at least one silicone oil, at least one ester that is prepared from of a straight chain or branched alcohol containing 1 to 17 carbon atoms and a straight chain or branched fatty acid containing 3 to 24 carbon atoms, and at least one liquid glycerine triester of branched or linear, saturated or unsaturated C₆ - C₂₂ fatty acids.

11. Composition according to one of the preceding claims, **characterised in that** the emulsifiers and/or surfactants are selected from
- addition products of 4 to 150 moles of ethylene oxide and/or 0 to 5 moles propylene oxide to linear fatty alcohols containing 8 to 22 carbon atoms or to fatty acids containing 12 to 22 carbon atoms in the alkyl group,
- C₁₂-C₂₂ fatty acid mono and diesters of addition products of 1 to 150 moles of ethylene oxide on polyols containing 3 to 6 carbon atoms, especially on glycerine,
- C₈-C₂₂ alkyl mono and oligoglycosides and their ethoxylated analogues,
- addition products of 5 to 150 moles of ethylene oxide on castor oil or hydrogenated castor oil,
- partial esters of polyols containing 3 to 6 carbon atoms with saturated fatty acids containing 8 to 22 carbon atoms,
- fatty acid esters of sugars and sugar alcohols such as sorbitol which can be optionally alkoxylated with 4 to 150 moles of ethylene oxide and/or 0 to 5 moles of propylene oxide.
- phospholipids.

12. Composition according to one of the preceding claims, **characterised in that** the at least one thickening polymer is selected from polyethylene glycols, polypropylene glycols, EO-PO-polyalkylene glycols and polyvinyl pyrrolidone, each with an average molecular weight of at least 1000 D and particularly preferably from 5000 to 50 000 D, starch, guar, cellulose ethers, hydroxyalkyl celluloses such as hydroxyethyl cellulose, hydroxypropyl cellulose, hydroxypropyl methyl cellulose, methyl cellulose, hydroxymethyl cellulose and carboxymethyl cellulose.

13. Composition according to one of the preceding claims, **characterised in that** at least one water-soluble polyol is comprised, selected from water-soluble diols, triols and higher hydric alcohols as well as polyethylene glycols that consist only of ethylene oxide units.

14. Composition according to one of the preceding claims, **characterised in that** at least one salt is comprised, selected from tripotassium phosphate, dipotassium hydrogen phosphate, potassium dihydrogen phosphate, sodium chloride and potassium chloride, in a total amount of 0.1 - 5 wt %, particularly preferably 0.2 - 3 wt % and exceptionally preferably 1 - 2 wt %, each relative to the total composition.

15. Non-therapeutic, cosmetic use of a composition according to one of claims 1 - 14 for the care, for cleaning and/or removing makeup from the skin, the mucosa, the eyes and/or the hair.

16. Non-therapeutic, cosmetic method for the care, for cleaning and/or removing makeup from the skin, the mucosa, the eyes and/or the hair, **characterised in that** a composition according to one of claims 1 - 14 is applied onto skin, mucosa, eyes and/or hair.

17. Method according to claim 16, **characterised in that** a composition according to one of claims 1 - 14 is applied onto the skin with a medium, cloth, pad, mop, flannel, sponge or netball.

18. Method according to claim 16 or 17, characterised that the composition according to one of claims 1 - 14 is rinsed off and or wiped off after the application.

## Revendications

1. Composition aqueuse liquide, à au moins deux phases, contenant au moins une huile et au moins un émulsifiant ou au moins un agent tensioactif, qui présente au moins une phase aqueuse continue I est au moins une phase huileuse discontinue II, la phase huileuse II formant, au repos, ensemble avec une partie d'une phase aqueuse I une couche d'émulsion, et une autre partie de la phase aqueuse I formant une couche aqueuse de la composition et la composition pouvant être transformée de manière réversible, temporairement en une émulsion par une agitation mécanique, **caractérisée en ce que**
a) la phase huileuse II contient au moins une huile de silicone liquide aux conditions normales,
b) la teneur en eau vaut 50-85% en poids, de préférence 60-75% en poids et de manière particulièrement préférée 65-70% en poids, à chaque fois par rapport à la totalité de la composition,
c) la teneur totale en émulsifiant et/ou en agent tensioactif est de 0,01 à moins de 0,1% en poids, à chaque fois par rapport à la totalité de la composition,
d) au moins un polymère épaississant est contenu en une quantité totale de 0,1-2% en poids, de préférence de 0,3-1% en poids et de manière particulièrement préférée de 0,3-0,5% en poids, à chaque fois par rapport à la composition totale.

2. Composition selon la revendication 1, **caractérisée en ce que** ladite au moins une huile de silicone liquide est choisie parmi les silicones de formule (CH₃)₃Si-[O-Si(CH₃)₂]ₓ-O-Si(CH₃)₃, dans laquelle x représente un nombre de 0 à 50, de préférence de 1 à 20 et en particulier de 3 à 10, et/ou les silicones de formule 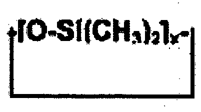 dans laquelle x représente un des nombres 3, 4, 5 ou 6.

3. Composition selon l'une quelconque des revendications précédentes, **caractérisée en ce que** ladite au moins une huile de silicone liquide est contenue en une quantité totale de 1-40% en poids, de préférence de 5-30% en poids, de manière particulièrement préférée de 10-25% en poids, de manière extrêmement préférée de 15-20% en poids, à chaque fois par rapport à la totalité de la composition.

4. Composition selon l'une quelconque des revendications précédentes, **caractérisée en ce que** la phase huileuse II contient en outre au moins un autre composant huileux liquide, qui n'est pas une huile de silicone, ni un parfum.

5. Composition selon l'une quelconque des revendications précédentes, **caractérisée en ce que** la phase huileuse II contient, comme autre composant huileux liquide, au moins un monoester, un diester ou un polyester d'un alcool linéaire ou ramifié, monovalent ou polyvalent, comprenant 1 à 17 atomes de carbone et d'un acide gras linéaire ou ramifié comprenant 3 à 24 atomes de carbone.

6. Composition selon la revendication 5, **caractérisée en ce que** l'ester est choisi parmi l'adipate de diisopropyle, l'adipate de di-n-butyle, l'hexanoate de 2-éthylhexyle, le laurate d'éthyle, le myristate de méthyle, le néopentanoate d'isodécyle, le myristate d'éthyle, le propionate de myristyle, le 2-éthylhexanoate de 2-éthylhexyle, l'octanoate de 2-éthylhexyle, le caprate/caprylate de 2-éthylhexyle, le palmitate de méthyle, le myristate de butyle, le myristate d'isobutyle, le palmitate d'éthyle, le laurate d'isohexyle, le laurate d'hexyle, le néopentanoate d'isodécyle, le néopentanoate d'isotridécyle, le néopentanoate d'isostéaryle, le néopentanoate d'octyldodécyle, l'isononanoate d'isononyle, l'isononanoate d'octyle, l'isononanoate d'isodécyle, l'isononanoate d'isotridécyle, l'isononanoate d'isostéaryle, le myristate d'isopropyle, le palmitate d'isopropyle, le stéarate d'isopropyle, l'isostéarate d'isopropyle, l'octanoate de cétyle, l'octanoate de tridécyle, le diheptanoate de PEG-4 et le palmitate de 2-éthylhexyle, le benzoate de C₁₂₋C₁₅-alkyle, le diheptanoate de néopentylglycol, le diéthyl-2-hexanoate de propylèneglycol, le 2-éthylhexanoate de 2-éthylhexyle, le triheptanoate de glycéryle ou leurs mélanges.

7. Composition selon l'une quelconque des revendications 5 à 6, **caractérisée en ce que** ledit au moins un autre composant huileux liquide, qui est un monoester, un diester ou un polyester d'un alcool linéaire ou ramifié, monovalent ou polyvalent, comprenant 1 à 17 atomes de carbone et d'un acide gras linéaire ou ramifié comprenant 3 à 24 atomes de carbone, est contenu en une quantité totale de 0,1-5% en poids, de préférence de 0,5-3% en poids, de manière particulièrement préférée de 1-2% en poids, à chaque fois par rapport à la totalité de la composition.

8. Composition selon l'une quelconque des revendications précédentes, **caractérisée en ce que** la phase huileuse II contient, comme autre composant huileux, liquide, au moins un triester de glycérol, liquide aux conditions normales, d'acides gras ramifiés ou linéaires, saturés ou insaturés, en C₆-C₂₂.

9. Composition selon la revendication 8, **caractérisée en ce que** ledit au moins un triester de glycérol d'acides gras ramifiés ou linéaires, saturés ou insaturés en C₆-C₂₂ est contenu en une quantité totale de 0,1-5% en poids, de préférence de 0,5-3% en poids, de manière particulièrement préférée de 1-2% en poids, à chaque fois par rapport à la totalité de la composition.

10. Composition selon l'une quelconque des revendications précédentes, **caractérisée en ce que** la phase huileuse II contient au moins une huile de silicone, au moins un ester, qui est préparé à partir d'un alcool linéaire ou ramifié, comprenant 1 à 17 atomes de carbone et d'un acide gras linéaire ou ramifié comprenant 3 à 24 atomes de carbone, et au moins un triester de glycérol liquide d'acides gras ramifiés ou linéaires, saturés ou insaturés en C₆-C₂₂.

11. Composition selon l'une quelconque des revendications précédentes, **caractérisée en ce que** les émulsifiants et/ou les agents tensioactifs sont choisis parmi :
- les produits d'addition de 4 à 150 moles d'oxyde d'éthylène et/ou de 0 à 5 moles d'oxyde de propylène sur des alcools gras linéaires comprenant 8 à 22 atomes de carbone ou sur des acides gras comprenant 12 à 22 atomes de carbone dans le groupe alkyle,
- les monoesters et diesters d'acides gras en C₁₂ à C₂₂ de produits d'addition de 1 à 150 moles d'oxyde d'éthylène sur des polyols comprenant 3 à 6 atomes de carbone, en particulier sur le glycérol,
- les (alkyle en C₈ à C₂₂)monoglycosides et les (alkyle en C₈ à C₂₂)oligoglycosides et leurs analogues éthoxylés,
- les produits d'addition de 5 à 150 moles d'oxyde d'éthylène sur de l'huile de ricin ou de l'huile de ricin durcie,
- les esters partiels de polyols comprenant 3 à 6 atomes de carbone avec des acides gras saturés comprenant 8 à 22 atomes de carbone,
- les esters d'acides gras de sucres et d'alcools de sucre tels que le sorbitol, qui peuvent le cas échéant être alcoxylés par 4 à 150 moles d'oxyde d'éthylène et/ou 0 à 5 moles d'oxyde de propylène,
- les phospholipides.

12. Composition selon l'une quelconque des revendications précédentes, **caractérisée en ce que** ledit au moins un polymère épaississant est choisi parmi les polyéthylèneglycols, les polypropylèneglycols, les OE-OP-polyalkylèneglycols et la polyvinylpyrrolidone, présentant à chaque fois un poids moléculaire moyen d'au moins 1000 D et de manière particulièrement préférée de 5000 à 50 000 D, l'amidon, la gomme guar, les éthers de cellulose, les hydroxyalkylcelluloses tels que l'hydroxyéthylcellulose, l'hydroxypropylcellulose, l'hydroxypropylméthylcellulose, la méthylcellulose, l'hydroxyméthylcellulose et la carboxyméthylcellulose.

13. Composition selon l'une quelconque des revendications précédentes, **caractérisée en ce qu'**au moins un polyol soluble dans l'eau, choisi parmi les diols, les triols et les alcools supérieurs solubles dans l'eau ainsi que les polyéthylèneglycols, qui sont uniquement constitués d'unités d'oxyde d'éthylène, est contenu.

14. Composition selon l'une quelconque des revendications précédentes, **caractérisée en ce qu'**au moins un sel, choisi parmi le phosphate tripotassique, l'hydrogénophosphate dipotassique, le dihydrogénophosphate potassique, le chlorure de sodium et le chlorure de potassium, en une quantité totale de 0,1-5% en poids, de manière particulièrement préférée de 0,2-3% en poids et de manière extrêmement préférée de 1-2% en poids, à chaque fois par rapport à la totalité de la composition, est contenu.

15. Utilisation non thérapeutique, cosmétique d'une composition selon l'une quelconque des revendications 1-14 pour le soin, le nettoyage et/ou le démaquillage de la peau, des muqueuses, des yeux et/ou des cheveux.

16. Procédé non thérapeutique, cosmétique pour le soin, le nettoyage et/ou le démaquillage de la peau, des muqueuses, des yeux et/ou des cheveux, **caractérisé en ce qu'**une composition selon l'une quelconque des revendications 1 - 14 est appliquée sur la peau, les muqueuses, les yeux et/ou les cheveux.

17. Procédé selon la revendication 16, **caractérisé en ce qu'**une composition selon l'une quelconque des revendications 1 - 14 est appliquée sur la peau à l'aide d'un support, d'un tissu, d'une compresse, d'un tampon, d'un chiffon, d'une éponge ou d'une éponge en filet.

18. Procédé selon la revendication 16 ou 17, **caractérisé en ce que** la composition selon l'une quelconque des revendications 1 - 14 est éliminée par rinçage et/ou essuyage après l'application.
